# EUROPÄISCHE PATENTSCHRIFT

(11) **EP 0 907 885 B1**
(45) Veröffentlichungstag und Bekanntmachung des Hinweises auf die Patenterteilung: **03.09.2003**
(21) Anmeldenummer: 97930400.3
(22) Anmeldetag: 25.06.1997
(51) Int. Cl.: G01N 33/50

(54) **VERFAHREN ZUM VERGLEICHENDEN SCREENEN VON SUBSTANZEN MIT PHARMAKOLOGISCHER WIRKUNG**
METHOD OF COMPARATIVE SCREENING OF PHARMACOLOGICALLY ACTIVE SUBSTANCES
PROCEDE DE CRIBLAGE COMPARATIF DE SUBSTANCES PHARMACOLOGIQUEMENT ACTIVES

(30) Priorität: 28.06.1996 EP 96110459
(43) Veröffentlichungstag der Anmeldung: 14.04.1999
(73) Patentinhaber: BOEHRINGER INGELHEIM INTERNATIONAL GmbH, 55218 Ingelheim am Rhein (DE)
(72) Erfinder: CZERNILOFSKY, Armin, Peter, A-2500 Baden (AT); VON RÜDEN, Thomas, A-2500 Baden (AT); HIMMLER, Adolf, A-1236 Wien (AT); LOEBER, Gerhard, A-2351 Wiener Neudorf (AT); METZ, Thomas, A-1010 Wien (AT); SCHNITZER, Renate, A-1230 Wien (AT); SPEVAK, Walter, A-2105 Oberrohrbach (AT); STRATOWA, Christian, A-1010 Wien (AT); TONTSCH, Ulrike, A-1170 Wien (AT); WEYER-CZERNILOFSKY, Ulrike, A-2500 Baden (AT); WICHE-CASTANON, Maria, Josefa, A-1130 Wien (AT)
(74) Vertreter: Laudien, Dieter, Dr.
(86) Internationale Anmeldenummer: EP9703329
(87) Internationale Veröffentlichungsnummer: WO98000713

(56) Entgegenhaltungen:
- WO-A-92/13063
- WO-A-93/11257

## Beschreibung

Die Erfindung bezieht sich auf ein Verfahren zum Screenen von Substanzen mit pharmakologischer Wirkung.

Screeningverfahren mit hoher Durchsatzrate ("High Throughput Screening") und verwandte Screening-Technologien sind in der pharmazeutischen Industrie zu den wichtigsten Verfahren für das Auffinden neuer Wirkstoffe geworden. Allgemein sind Verfahren dieser Art dadurch definiert, daß eine große Zahl von Substanzen, die von Reinchemikalien-Banken, natürlichen Produkten oder aus der kombinatorischen Chemie stammen, in spezifischen biologischen Assays, die im Hinblick auf die erwünschte Wirkung des Arzneimittels entworfen wurden, in kürzestmöglicher Zeit auf kostengünstigste Art getestet werden. Konventionelle Assay-Typen, z.B. Rezeptor-Bindungs- oder Enzymsubstrat-Assays, wurden bereits auf relativ einfache Weise modifiziert, um im High Throughput-Format zu arbeiten, was durch die Fortschritte bei den Geräten, z.B. die Methoden der Handhabung auf Flüssigbasis, und die Nachweismethoden, in Vereinigung mit Robotersystemen bedingt ist.

Assay-Systeme auf der Grundlage lebender Zellen wurden bisher in High Throughput Screens weniger häufig eingesetzt, vor allem aufgrund der bei der Standardisierung auftretenden Schwierigkeiten, der Schwierigkeit bei der Interpretation der Ergebnisse aus dem primären Screening und der Unzulänglichkeit der Geräte bei der Bewältigung der Anforderungen von zellulären Systemen und langer Inkubationszeiten. Ein weiterer Grund dafür, warum der Einsatz zellulärer Assays für das High Throughput Screening bisher als weniger attraktiv angesehen wurde, ist in dem großen Aufwand gelegen, der an den primären Screen, d.h. an den ersten Screening-Durchgang, anschließt, um zwischen toxischen, nicht-spezifischen, oder nicht-selektiven Effekten einer Substanz zu unterscheiden bzw. um die Wirkungsmechanismen aufzuklären, die in der Zelle ablaufen, wenn das zelluläre System auf eine Substanz anspricht, also bei einem "Treffer".

Mit Hilfe der hochentwickelten Techniken der Molekularbiologie können biologische Zielmoleküle, deren Aktivität das zu identifizierende Arzneimittel modulieren soll, in eukaryotische Zellen, insbesondere Säugetierzellinien eingebaut werden. Außerdem stehen verschiedene Methoden zur Verfügung, um den gewünschten Effekt in der Zielzelle nachzuweisen, z.B. die Produktion eines neuen Proteins als Folge der Stimulierung eines bestimmten Rezeptors, die Aktivierung oder Zunahme von an den Zielmechanismus gekoppelten meßbaren Ereignissen, z.B. die Expression eines Reportergenproduktes wie Luciferase oder Green Fluorescent Protein (GFP), oder die Beeinflussung Schlüsselereignissen, die nach Rezeptoraktivierung in der Zelle auftreten, wie z.B. Apoptose.

Die Charakteristika eines guten Assays auf zellulärer Basis sind folgende:
a) Das "Signal/Noise"-Verhältnis, d.h. das Verhältnis von maximaler Änderung und Normalwert des Meßsignals, muß ein günstiges sein, d.h. das Hintergrund-Signal sollte niedrig genug sein, um eine Induktion des zu messenden Ereignisses mit hoher Empfindlichkeit zu erfassen, andererseits aber hoch genug, um die Nachweisgrenze im Hinblick auf die Negativkontrolle bestimmen zu können. b) Die gemessenen Signale müssen über einen gewissen Zeitraum reproduzierbar und stabil sein. c) Die Test-Zellen müssen relativ einfach zu züchten und widerstandsfähig gegenüber der Behandlung im Assay sein. d) Das in die Zellen eingeführte biologische Zielmolekül und die daran gekoppelten Regulationsmechanismen müssen über einen längeren Zeitraum (einige Monate) stabil sein. e) Die Eigenschaften der Zelle müssen über einen längeren Zeitraum gleich sein; d.h. die verwendeten Zellinien als solche müssen ebenfalls stabil sein.

Die Erfüllung dieser Anforderungen stellt die Grundlage für ein Screening-System dar, dessen Vorteil vor allem darin besteht, daß lebende Zellen ein Substrat bereitstellen, das den zu testenden Substanzen das Zielmolekül, das sie als potentielle Therapeutika letztlich im lebenden Organismus modulieren sollen, in der biologisch richtigen Umgebung anbietet. Ein Beispiel für ein Testsubstrat sind z.B. Zellen einer Zellinie, die einen klonierten Rezeptor und den damit assoziiierten Signalübertragungsweg, gekoppelt an ein passendes Reportersystem enthalten.

Eine hohe Effizienz von High Throughput-Strategien ist dann gegeben, wenn es gelingt, die im Primärscreen anfallende Information zu maximieren. Das bedeutet gleichzeitig, daß der Aufwand im Anschluß an den Primär-Screen, der darin besteht, die erhaltenen Treffer in qualitativer Hinsicht zu differenzieren, möglichst minimiert wird. Bei den heute möglichen Screening-Durchsatzraten, die in der Größenordnung von einer Million Substanzen pro Jahr liegen, bringt eine Trefferrate von mehr als 1% einen unannehmbaren Folgeaufwand mit sich. Typische Trefferquoten von mehr als 1% und die damit verbundenen aufwendigen Sekundärassays haben zur Folge, daß trotz der prinzipiellen Vorteile zellulärer Assays häufig noch immer auf alternative Screening-Formate, wie Rezeptor-Bindungs- oder Enzymsubstrat-Assays, ausgewichen wird.

Die der vorliegenden zugrundeliegende Aufgabe besteht darin, die grundsätzlich bestehenden Vorteile von zellulären Assays zu maximieren und ein Verfahren bereitzustellen, das die bei zellulären Assays im High Throughput-Format auftretenden Nachteile beseitigt.

Der Begriff des "Parallelscreening" wird im allgemeinen auf den logistischen Mechanismus beim Screening angewendet, wobei mehrere verschiedene Assays bzw. Assay-Formate, unter Kontrolle eines Roboters, mit derselben Geräteanordnung ausgeführt werden.

Der vorliegenden Erfindung lag die Überlegung zugrunde, diesen Ansatz für ein vergleichendes Screening weiterzuentwickeln, indem aus demselben Substanzvorrat mehrere unterschiedliche zelluläre Assayformate gleichzeitig beschickt werden.

Die vorliegende Erfindung betrifft ein Verfahren zum Bestimmen der pharmakologischen Wirkung einer Substanz auf die Aktivität unterschiedlicher biologischer Zielmoleküle durch Aufbringen der Substanz auf Testzellen, die ein oder mehrere biologische Zielmoleküle enthalten und Bestimmung der Wirkung der Substanz auf die Aktivität der Zielmoleküle. Das Verfahren ist dadurch gekennzeichnet, daß man eine definierte Menge einer Testsubstanz in einem Arbeitsschritt
a) auf Testzellen mit derselben biologischen Grundausstattung aufbringt, die sich dadurch unterscheiden, daß sie ein oder mehrere unterschiedliche biologische Zielmoleküle enthalten; und/oder
b) auf Testzellen aufbringt, die ein oder mehrere biologische Zielmoleküle enthalten, wobei sich die Zellen dadurch unterscheiden, daß sie unterschiedliche biologische Grundausstattung aufweisen, und daß man
   i) die Wirkung der Substanz auf das bzw. die biologischen Zielmoleküle mittels eines an die Aktivierung des Zielmoleküls gekoppelten Nachweissystems mißt; und/oder
   ii) die Wirkung der Substanz auf unterschiedliche durch die Aktivierung des Zielmoleküls ausgelöste Regulationsmechanismen bestimmt, indem man die Wirkung mittels mehrerer, jeweils an die unterschiedlichen Regulationsmechanismen gekoppelten Nachweissysteme mißt,
und daß man die Wirkungen der Testsubstanz auf die unterschiedlichen Testzellen bzw. die mittels unterschiedlicher Nachweismethoden ermittelten Wirkungen unmittelbar miteinander vergleicht.

Die im erfindungsgemäßen Verfahren eingesetzten Testzellen, die ein oder mehrere bestimmte biologische Zielmoleküle enthalten, stellen ein definiertes zelluläres Substrat dar, auf das die jeweilige Testsubstanz aufgebracht wird. Das Substrat (im Anwendungsfall a) Zellen mit identischem biologischem Hintergrund und unterschiedlichen Zielmolekülen; im Fall b) Zellen mit unterschiedlichem biologischem Hintergrund (Grundausstattung) und identischem Zielmolekül) besteht im allgemeinen aus einer Anzahl (bis ca. 10⁵) identischer Zellen, die sich jeweils in einer Vertiefung einer Kulturplatte befinden. Gegebenenfalls können auch Einzelzellen das Substrat darstellen und mit der Substanz beaufschlagt werden, wenn das Nachweissystem empfindlich genug ist, z.B. wenn im Fall der Messung optischer Signale die apparativen Voraussetzungen gegeben sind, die Signale auf physikalischem Weg entsprechend zu verstärken.

Das wesentliche Merkmal der Erfindung besteht darin, daß in einem Arbeitsschritt auf eine Anzahl verschiedener zellulärer Substrate dieselbe Substanz aufgebracht wird. Jedes Substrat repräsentiert einen individuellen Assay-Typ. Wenn unterschiedliche Nachweissysteme eingesetzt werden, um die Wirkung der Testsubstanz auf unterschiedliche Regulationsmechanismen desselben Zielmoleküls zu untersuchen, liegen unterschiedliche Assay-Formate auf der Grundlage desselben Substrats Vor.

Das erfindungsgemäße Verfahren hat den Vorteil, durch Verwendung verschiedener Assays bzw. Assay-Formate bei gleichzeitigem Einsatz identischer Logistik (identische Substanzquelle) die für die Auswertung maßgeblichen Variablen auf ein Minimum zu reduzieren. Außerdem hat das Vefahren den Vorteil, daß die in den verschiedenen Assays in einem Arbeitsgang erhaltenen Ergebnisse nach strengen Maßstäben verglichen und ausgewertet werden können.

Der Arbeitsschritt, in dem eine definierte Menge einer Testsubstanz auf die Zellen aufgebracht wird, wird im Rahmen des High Throughput Screens im allgemeinen auf eine Anzahl von Substanzen ausgedehnt, d.h. es werden mehrere Substanzen, gegebenenfalls jeweils in mehreren Verdünnungen, parallel auf eine oder mehrere Batterien von zellulären Substraten, wobei jede Batterie eine Gruppe unterschiedlicher Assays bzw. Assay-Formate auf der Grundlage derselben Ausgangszelle darstellen, aufgebracht.

Nach gegebenenfalls an den Primärscreen anschließenden weiteren Screeningschritten, in denen die Testsubstanz(en) auf eine weitere Batterie von zellulären Substraten aufgebracht wurde, können die erhaltenen Treffer in eine Anzahl von Klassen eingeteilt werden, z.B. nach Spezifität ihrer Wirkung auf nur einen Zelltyp, Nicht-Spezifität in allen eingesetzten Zelltypen, oder Spezifität in einigen, aber nicht allen gestesteten Zelltypen, nach ihrer Spezifität auf nur einen an das Zielmolekül gekoppelten Signalübertragungsweg.

Bei den Zellen handelt es sich im allgemeinen um Säugetierzellen, insbesondere humane Zellen; es können jedoch auch andere eukaryotische Zellen, insbesondere Hefezellen, als Testzellen verwendet werden.

Als Ausgangszellen für die Testzellen werden zweckmäßig Zellen verwendet, die leicht kultivierbar sind und über einen stabilen Genotyp verfügen. Es können Zellen verwendet werden, die das interessierende Zielmolekül endogen exprimieren. Vorzugsweise werden jedoch Zellen eingesetzt, welche die interessierenden Zielmoleküle von sich aus gar nicht oder nur schwach exprimieren. Die Zellen werden mittels üblicher Transfektionsmethoden mit der für das biologische Zielmolekül kodierenden DNA, z.B. mit Rezeptor-DNA, transformiert (vgl. z.B. Potter et al. 1984; Felgner et al., 1987), bevorzugt werden die Elektroporations-, die Calciumphosphat-Präzipitationsoder die Lipofektionsmethode eingesetzt.

Die Zellen dienen einerseits für die Herstellung von Testzellen, indem sie das Ausgangssubstrat für die Aufnahme der für das Zielmolekül kodierenden DNA darstellen, andererseits werden sie als Kontrollzellen eingesetzt, um zu überprüfen, ob ein mittels eines Nachweissystems gemessenes Signal tatsächlich auf die Wirkung der Testsubstanz zurückzuführen ist. Erzeugt die Substanz in der Testzelle ein Signal und in der als Kontrollzelle verwendeten Ausgangszelle nicht, so ist die über das Signal festgestellte Wirkung auf die Testsubstanz zurückzuführen. Wenn die Kontrollzelle ebenfalls ein Signal gibt, beeinflußt die Substanz (auch) einen zellulären Prozeß, der unabhängig von der Aktivierung des Zielmoleküls ist; der diesem Signal entsprechende Kontroll-Meßwert ist bei der Auswertung der Messung zu berücksichtigen.

Im Hinblick auf die möglichst einfache Durchführung der jeweils eingesetzten Meßmethode werden Zellen verwendet, die dafür die geeigneten Voraussetzungen mitbringen. Für Testzellen, bei denen Proliferation bzw. Apoptose gemessen wird, können sowohl adhärierende Zellen als auch Zellen, die in Suspension wachsen, verwendet werden, deren Überleben in Kultur strikt von der Anwesenheit bestimmter Wachstumsfaktoren abhängig ist. Werden diese Wachstumsfaktoren entzogen, stellen die Zellen das Wachstum ein oder leiten binnen weniger Stunden Apoptose ein. Für Testzellen, die auf der Messung der Genexpression basieren, werden vorzugsweise gut adhärierende Zellen verwendet, zum Beispiel Fibroblasten, Epithelzellen, Endothelzellen, etc., weil diese Meßmethode einen Waschschritt erfordert.

Wenn entsprechend Ausführungsform a) des erfindungsgemäßen Verfahrens Zellen mit derselben biologischen Grundausstattung verwendet werden, handelt es sich dabei Zellen desselben Typs, also um Zellen, die aus einem bestimmten Gewebe isoliert wurden und u.a. durch molekularbiologische, morphologische, biochemische und immunologische Parameter definiert sind. Vorzugsweise werden als Testzellen klonale Zellen verwendet, im allgemeinen Zellen einer permanenten Zellinie. Diese Zellen stammen von einer einzelnen Ursprungszelle ab und haben einen identischen Genotyp.

Für die Ausführungsform der Erfindung gemäß b) sind Zellen mit unterschiedlicher biologischer Grundausstattung definitionsgemäß nicht nur Zellen unterschiedlichen Typs, sondern auch Zellen desselben Typs, aber unterschiedlichen Differenzierungs- oder Aktivierungszustandes. Auf diese Weise kann z.B. der Einfluß einer Testsubstanz auf normale Zellen mit ihrer Wirkung auf Tumorzellen, die aus demselben Gewebe stammen, verglichen werden, oder es kann die Wirkung der Substanz auf Zellen einer etablierten Zellinie mit der Wirkung auf Primärzellen desselben Ursprungs verglichen werden, was eine Beurteilung der Aussagekraft eines Screens auf der Grundlage von permanenten Zellinien ermöglicht.

Biologische Zielmoleküle sind jene Moleküle, deren biologische Aktivität durch die Testsubstanz beeinflußt werden soll, um eine aus dieser Aktivität resultierende unerwünschte Reaktion der Zelle, die für einen pathologischen Effekt verantwortlich ist, zu blockieren.

Grundsätzlich kommen als Zielmoleküle sämtliche natürlicherweise in Säugetierzellen, insbesondere menschlichen Zellen, vorkommenden Proteine bzw. Fragmente oder Mutanten davon in Betracht, die konstitutiv aktiv sind bzw. deren Aktivierung durch ein extrazelluläres Signal (den natürlichen Liganden), eine Reaktion der Zelle herbeiführt, die in irgendeiner Form direkt oder indirekt meßbar ist. Die bei Inhibierung durch eine Testsubstanz auftretende Beeinflussung dieser Reaktion ist somit ebenfalls meßbar, was beim Einsatz des Verfahrens als robotergesteuertes High Throughput Verfahren eine automatische Auswertung ermöglicht.

Biologische Zielmoleküle sind Komponenten verschiedener zellulärer, u.a. metabolischer oder Rezeptorgekoppelter, Signalübertragungswege, wobei diese Komponenten grundsätzlich von jedem beliebigen Abschnitt der Signalübertragungskaskade stammen können.

Beispiele für biologische Zielmoleküle sind Vertreter der großen Gruppe der Rezeptoren, insbesondere Rezeptoren für Wachstums- und Differenzierungsfaktoren einschließlich deren Subtypen. Beispiele für Rezeptoren sind Rezeptortyrosinkinasen (EGF-Rezeptor, PDGF-Rezeptor, etc.); Serin/Threonin-Kinasen, wie der TGF-β-Rezeptor; Integrinrezeptoren; Rezeptoren der Klasse LIF, Oncostatin M, CNTF, etc. (gp130); Rezeptorphosphatasen; Rezeptoren für Zytokine (Interleukine, Interferone etc.) und G-Protein-gekoppelte Rezeptoren, die an den Phospholipase C-Signalübertragungsweg oder den Adenylatzyklase-Signalübertragungsweg koppeln können, z.B. Neuropeptid Y-Rezeptoren (auch PYY, NPP) oder Neurokinin-Rezeptoren (NK_{1,2,3}).

Ein Verfahren zum Screenen von Substanzen mit modulierender Wirkung auf rezeptorabhängige zelluläre Signalübertragungswege und dessen Anwendung auf verschiedene Rezeptoren ist z.B. in der WO 93/11257 beschrieben, auf deren Offenbarung hiermit Bezug genommen wird. Das Prinzip des Verfahrens beruht auf der Verwendung von Testzellen, die ein Reportergen unter der Kontrolle einer regulatorischen Sequenz, die auf die Änderung der Konzentration eines Botenstoffes eines rezeptorabhängigen Signalübertragungsweges anspricht ("Sensor-DNA"), sowie die interessierende Rezeptor-DNA enthalten. Wird die Testsubstanz auf die Testzellen aufgebracht, äußert sich die Wirkung der Testsubstanz auf das Zielmolekül, also den interessierenden Rezeptor bzw. ein Element des an diesen Rezeptor gekoppelten Signalübertragungsweges, in einer Änderung der Expression des Reportergens. Gemäß Ausführungsform a) in Verbindung mit i) des erfindungsgemäßen Verfahrens werden für ein vergleichendes Screening auf der Grundlage dieses Assay-Prinzips identische Ausgangszellen mit identischem Reportergen-Konstrukt ("Sensor-DNA") und mit verschiedener Rezeptor-DNA transformiert, was einen direkten Vergleich der Wirkung der Testsubstanz auf die Aktivierung von Signalübertragungswegen unterschiedlicher Rezeptoren bzw. Rezeptorsubtypen ermöglicht.

Gemäß Variante b) in Verbindung mit i) werden Zellen unterschiedlichen Typs mit identischer Rezeptor-DNA und identischer Sensor-DNA transformiert, um die Wirkung der Testsubstanz auf den an den jeweiligen Rezeptor gekoppelten Signalübertragungsweg in verschiedenen Zelltypen zu untersuchen. Dies ist vor allem dann von Interesse, wenn man ein Arzneimittel mit Selektivität für einen bestimmten Zelltyp sucht, d.h. wenn die Substanz den Signalübertragungsweg nur in einem bestimmten Zelltyp beeinflussen soll, in anderen Zelltypen jedoch nicht, beispielsweise den Signalübertragungsweg des IL-5-Rezeptors, der auf Eosinophilen und bestimmten T-Zellen, nicht jedoch auf anderen Zellen exprimiert wird.

Die Aktivierung eines Rezeptors bzw. Rezeptorsubtyps kann aufgrund komplexer intrazellulärer Wechselwirkungen die Aktivierung mehr als eines Signalübertragungsweges/Effektorsystems auslösen, z.B. wenn ein G-Protein-gekoppelter Rezeptor mit mehr als einer G-Protein-Variante wechselwirken kann. Beispielsweise koppelt der Angiotensin AT₁-Rezeptor an den Phospholipase C- und den Adenylatzyklase-Signalübertragungsweg, der AT₂-Rezeptor hingegen an das Effektorsystem des zyklischen GMP. Ein weiteres Beispiel ist der Prostanoid-Rezeptor EP₃, der an den Phospholipase C- und den Adenylatzyklase-Signalübertragungsweg koppelt, während andere Prostanoidrezeptoren nur an den Adenylatzyklase-Signalübertragungsweg koppeln bzw., in einem Fall, ein nukleärer Rezeptor (PPARγ) direkt aktiviert wird. Wenn die Ursache für pathologische Veränderungen der Zelle in einer Störung dieser Wechselwirkungen gelegen ist, kann es von Interesse sein, Arzneimittel zu finden, die spezifisch auf nur eines der Effektorsysteme einwirken. In diesem Fall kann die Ausführungsform des Verfahrens gemäß a) in Verbindung mit ii) eingesetzt werden. Wenn z.B. der eine Signalübertragungsweg das Phospholipase C-Effektorsystem, bei dessen Aktivierung die Botenstoffe Inositol-1,4,5-Triphosphat (IP₃) und Diacylglyzerin (DAG) gebildet werden, und der andere das Adenylatzyklase-Effektorsystem ist, bei dem der Botenstoff cAMP gebildet wird, können zwei Testzellen, transformiert mit demselben Rezeptor, eingesetzt werden, die sich durch die Sensor-DNA unterscheiden, wobei die eine auf die IP3/DAG-Konzentration und die andere auf die cAMP-Konzentration anspricht. Beispiele für geeignete Sensor-DNA-Konstrukte sind der WO 93/11257 zu entnehmen. Die Erfindung ermöglicht in diesem Fall in einem Screening-Durchgang eine direkte Aussage darüber, ob eine Substanz Spezifität für nur einen Signalübertragungsweg hat.

Es können auch in einem Screening-Durchgang die Varianten ai) und aii) kombiniert werden.

Weitere Beispiele für Zielmoleküle sind intrazelluläre Komponenten von Signalübertragungswegen, z.B. Proteinkinasen (Src, Raf, MAPK, JAK), Adaptormoleküle (GRB, Sos) oder andere Elemente von Signalübertragungswegen, wie Ras.

Zielmoleküle können auch weiter stromabwärts im Signalübertragungsweg gelegene Komponenten sein, wie Elemente der induzierbaren Genexpression (Ligandenregulierte Transkriptionskontrolle), insbesondere Transkriptionsfaktoren, z.B. Myc, Fos, Myb, NF-κB, AP-1, STAT, Ets-Proteine und/oder deren Co-Faktoren, oder Moleküle, die an der Zellapoptose beteiligt sind (Bcl-2, p53-Mutanten, oder die Apoptose regulierende Proteasen), ferner Kinasen, Phosphatasen oder GTPasen. Als Zielmoleküle können auch intrazelluläre Hormonrezeptoren, wie Steroidrezeptoren oder Retinoidrezeptoren, verwendet werden. Dem Fachmann sind derartige Elemente bekannt; eine Übersicht über die induzierbare Genexpression und die daran beteiligten Komponenten kann einschlägigen Fachbüchern entnommen werden, z.B. "Inducible Gene Expression", 1995.

Die Zielmoleküle können identisch mit den in der Zelle natürlicherweise vorkommenden Proteinen sein; es können aber auch in bestimmten Abschnitten mutierte oder hinsichtlich bestimmter Abschnitte gegenüber dem Wildtyp veränderte, z.B. verkürzte, Versionen dieser Proteine eingesetzt werden. Damit kann z.B. der Einfluß einer Testsubstanz auf bestimmte Domänen des Zielmoleküls untersucht werden, z.B. auf die Transaktivierungsdomäne oder auf einen Abschnitt des Proteins, der für die Wechselwirkung mit einem Co-Faktor verantwortlich ist.

Die Wirkung einer Testsubstanz auf das biologogische Zielmolekül wird von der Zelle als Signal wahrgenommen, das je nach Zelltyp zu unterschiedlichen Reaktionen der Zelle führt. Derartige Reaktionen, z.B. Änderung des intrazellulären Calcium-Spiegels, Phosphorylierung oder Dephosphorylierung verschiedener Proteine, Transkription bzw. Expression von Genen, DNA-Synthese, Zellteilung oder Zelldifferenzierung (morphologische Veränderungen, Expression von Markergenen), Apoptose, Chemotaxis, Zelladhäsion, sind dann unerwünscht, wenn sie für pathologische Zustände verantwortlich sind.

Mit Hilfe des erfindungsgemäßen Verfahrens ist es möglich, zelltypspezifisch bereits im primären Screen Testsubstanzen nach ihrer Wirkung auf solche unerwünschte Reaktionen zu identifizieren.

Prinzipiell lassen sich sämtliche der durch Aktivierung des biologischen Zielmoleküls ausgelösten zellulären Reaktionen mittels physikalisch-chemischer Methoden direkt oder indirekt bestimmen; der im Nachweissystem erhaltene Meßwert korreliert mit der Wirkung der Testsubstanzen auf das Zielmolekül.

Häufig resultiert die zelluläre Antwort auf Wachstumsfaktoren in der Zellteilung (Proliferation) oder in der Verhinderung des programmierten Zelltodes (Apoptose). Sowohl die Beeinflussung der Proliferation als auch der Apoptose als Folge der Wirkung der Testsubstanz lassen sich sehr leicht im Rahmen eines vollautomatisierten Screenings messen.

In einer bevorzugten Ausführungsform der Erfindung beruht das Verfahren auf der Messung der Proliferation.

Ein auf Proliferationsmessung basierendes Versuchssystem kann z.B. folgendermaßen aufgebaut sein: Zellen, die wachstumsfaktorabhängig proliferieren, werden vorgelegt, anschließend gibt man Verdünnungen der zu testenden Substanzen zu und inkubiert die Testzellen für einen bestimmten Zeitraum. Dann werden die Zellen mit einem Assayreagenz versetzt und nach einer weiteren kurzen Inkubation in einem Mikrotiterplattenphotometer gemessen. Dabei wird die Inkubationszeit so bemessen, daß Testzellen, denen der Wachstumsfaktor entzogen wurde, und Testzellen, die in Gegenwart von Wachstumsfaktor inkubiert werden, in diesem Zeitraum einen deutlichen Unterschied in der Zellzahl aufweisen.

Eine weitere im Rahmen der vorliegenden Erfindung bevorzugt verwendete Nachweismethode beruht auf der Messung der Expression von Reportergenen.

Ein Reportergen ist dadurch definiert, daß sein Expressionsprodukt durch Messung eines Signals, dessen Größe seiner Konzentration proportional ist, nachweisbar und quantifizierbar ist. Im Rahmen der vorliegenden Erfindung können sämtliche Reportergene eingesetzt werden, deren Expression mit hoher Empfindlichkeit in einem automatisierbaren Assay gemessen werden kann.

Beispiele für Reportergene sind das alkalische Phosphatase-Gen sowie das β-Galaktosidase- und das β-Glucuronidase-Gen; die von diesen Enzymen katalysierten Reaktionen werden mit Hilfe etablierter Fluoreszenz-Assays verfolgt (Wieland et al., 1985; Kricka, 1988), ferner Chloramphenicol-Acetyltransferase (CAT; Hartmann, 1991).

Ein im Rahmen der vorliegenden Erfindung bevorzugtes Reportergen ist das für Photinus pyralis-Luciferase (Firefly-Luciferase) kodierende Gen (De Wet et al., 1987). Dieses Enzym weist die Vorteile auf, daß es mit seinem Substrat Luciferin unter Zugabe von ATP Bioluminiszenz in hoher Ausbeute erzeugt, die mit Hilfe etablierter, automatisierbarer Methoden gemessen werden kann, und daß dieses Enzym von Säugetierzellen nicht endogen produziert wird. Ferner hat Luciferase eine relativ kurze *in vivo* Halbwertszeit und ist auch in hohen Konzentrationen nicht toxisch (Hartmann, 1991; Brasier et al., 1989). Die Messung der Aktivität der Firefly-Luciferase mittels Biolumineszenz stellt eine der empfindlichsten Methoden einer Enzymbestimmung dar. Aus diesem Grund und aufgrund des Fehlens von Luciferaseaktivität in normalen Säugetierzellen ist dieses Enzym als Reportergen besonders geeignet (Subramani und DeLuca, 1987). Ein weiteres bevorzugtes Reportergen ist das Green Fluorescent Protein (GFP; Inouye und Tsuji, 1994; Chalfie et al., 1994).

Reportergene können u.a. eingesetzt werden, um die durch Einwirkung der Testsubstanz ausgelöste Transkription von Genen nachzuweisen:

Die Stimulation mehrerer verschiedener Signalübertragungswege mündet in der Transkription einer Gruppe von Genen, z.B. der sog. "immediate early genes" wie c-fos, c-jun, c-myc. Über die Messung der Expression dieser Gene kann somit die Wirkung von Testsubstanzen auf die Aktivierung oder Inhibierung verschiedener Signalübertragungswege nachgewiesen werden.

Eine direkte Messung der Genexpression, z.B. mittels Antikörpern gegen die Expressionsprodukte, ist technisch aufwendig und im allgemeinen nicht für das automatisierte Screening anwendbar. Dagegen läßt sich die Expression ausgewählter Gene auf relativ einfachem Weg indirekt bestimmen. Dabei bestimmt man die Wirkung der Testsubstanz auf das Zielmolekül, indem man stellvertretend für die Expression des Original-Gens, also des Gens, das natürlicherweise am Ende der durch Aktivierung des Zielmoleküls ausgelösten intrazellulären Signalkaskade exprimiert wird, die Expression eines Reportergens (z.B. des Luciferase-Gens) mißt, das unter Kontrolle der regulatorischen Sequenz des Original-Gens (z.B. c-fos) steht. Die Expressionswerte für das Reportergen sind direkt proportional der Wirkung der Testsubstanz auf das Zielmolekül. Ein solches Screening-Verfahren auf der Grundlage der transkriptionellen Modulation, das sich für den Einsatz im High Throughput-Format eignet, ist in der WO 92/13063 beschrieben, auf deren Offenbarung hiermit Bezug genommen wird.

Die unterschiedlichen im Rahmen der vorliegenden Erfindung gleichzeitig in einem Arbeitsschritt oder nacheinander in nachfolgenden Screeningschritten eingesetzten Nachweissysteme können sich einerseits hinsichtlich des Nachweisprinzips selbst (z.B. Reportergen-Expression gegenüber Apoptose) und/oder im Aufbau des Nachweissystems unterscheiden. Dies erlaubt eine differentielle Aussage über die Wirkung einer Testsubstanz auf unterschiedliche Regulationsmechanismen, welche in unterschiedlichen, u.a. pathologischen, Wirkungen resultieren (z.B. Verwendung unterschiedlicher Sensor-DNA-Moleküle, welche auf unterschiedliche Effektorsysteme ansprechen oder Promotoren unterschiedlicher Zielgene enthalten). Damit eröffnet das erfindungsgemäße Verfahren die Möglichkeit der Differenzierung im Hinblick auf die gezielte Ausschaltung eines bestimmten unerwünschten biologischen Effekts. Will man z.B. eine Substanz finden, die die Proliferation eines bestimmten Zelltyps hemmt, nicht jedoch die Differenzierung, und man weiß, daß diese beiden Reaktionen in derselben Zelle durch Aktivierung unterschiedlicher Regulationsmechanismen desselben Zielmoleküls hervorgerufen werden, kann die Wirkung der Testsubstanz durch ein entsprechendes Assay-Design einem der beiden Signalübertragungswege zugeordnet werden. Dazu kann z.B. in einem vergleichenden Screen die Wirkung der Substanz auf denselben Zelltyp mit zwei unterschiedlichen Nachweissystemen, die jeweils auf die unterschiedlichen Regulationsmechanismen ansprechen, bestimmt werden. Wenn bekannt ist, daß die Aktivierung eines bestimmten Regulationssystems mit einer bestimmten, unerwünschten Reaktion der Zelle korreliert, z.B. wenn die Aktivierung eines bestimmten Signalübertragungsweges zur Apoptose führt, kann der Einfluß der Testsubstanz auf die Apoptose nicht nur direkt durch Messung der Apoptose, sondern auch indirekt über ein an diesen Signalübertragungsweg gekoppeltes Reportergen-System bestimmt werden. In diesem Fall zeugt die Expression des Reportergenproduktes als Folge der Einwirkung der Testsubstanz davon, daß die Substanz für den gewählten Zelltyp die Apoptose beeinflussen würde.

Das erfindungsgemäße Verfahren bietet ferner die Möglichkeit, auf raschem und effizientem Weg eine Aussage über ein Zielmolekül zu treffen, das bereits in klonierter Form vorliegt, dessen Funktion jedoch ganz oder teilweise unbekannt ist, z.B. über einen bestimmten Rezeptorsubtyp. Wenn eine Testzelle, die mit dem Rezeptor unbekannter Funktion transformiert ist, zusammen mit einer Serie von Testzellen identischer biologischer Grundausstattung, die andere Rezeptoren mit bekannter Funktion enthalten, in einem Assay eingesetzt werden, dessen Nachweissystem direkt bzw., indirekt über ein Reportergensystem, den Einfluß von Testsubstanzen auf eine biologische Wirkung erfaßt, erlaubt der Vergleich der Wirkung der Testsubstanzen auf die verschiedene Rezeptoren enthaltenden Testzellen eine Aussage über die Funktion des Rezeptors mit bisher unbekannter Funktion.

Im Rahmen der vorliegenden Erfindung wurden u.a. Zellen einer Wachstumsfaktor-abhängigen Zellinie mit unterschiedlichen funktionellen biologischen Zielmolekülen, deren Aktivierung im Tumorgeschehen eine Rolle spielt, transformiert und als Substrat in einem High Throughput Screen eingesetzt. Dabei wurde jede Gruppe von zellulären Testsubstraten, bestehend aus jeweils mit einem der biologischen Zielmoleküle transformierten Zellen, in mehrfacher Ausführung, jeweils mit einzelnen aus einer Serie von Testsubstanzen beaufschlagt und die Proliferation der Zellen gemessen. Als Zielmoleküle wurden der EGF (Epidermal Growth Factor)-Rezeptor, der HER2-Rezeptor, der Rezeptor (KDR) für den vaskulären Endothelzellwachstumsfaktor (VEGF), der MET-HGF (Hepatocyte Growth Factor)-Rezeptor sowie aktivierte Ras-Proteine verwendet.

Diese Zielmoleküle wurden aufgrund ihrer Bedeutung als potentielle Interventionsstellen im Rahmen von Tumortherapien gewählt:

Dem EGF-Rezeptor (EGF-R) wird eine wichtige Rolle bei der Entstehung diverser Tumoren epithelialen Ursprungs beigemessen. Dieser Rezeptortyp ist in Tumorgeweben häufig konstitutiv aktiviert, und zwar durch Überexpression, Genamplifikation, autokrine Stimulation der Mutationen. Aufgrund dieser Beobachtung ist der EGF-R ein potentiell wichtiger Angriffspunkt bei der Tumortherapie. Neben EGF-R-spezifischen monoklonalen Antikörpern und toxinbeladenen Liganden wurden bisher einige selektive Inhibitoren der EGF-R-assoziierten Tyrosinkinase beschrieben. Zur Suche nach neuen Inhibitoren des EGF-R wurden im Rahmen der vorliegenden Erfindung zelluläre Testsysteme auf der Grundlage von FDC-P1-Zellen entwickelt, die genetisch so verändert wurden, daß sie den funktionellen humanen EGF-R · exprimieren. Da die FDC-Pl-Zellen IL-3 abhängig sind, kann die Proliferation dieser Zellen entweder durch murines IL-3 oder durch EGF (der in Zellen, die den EGF-Rezeptor ektop exprimieren, IL-3 ersetzen kann) stimuliert werden (von Rüden, T. et al., 1988; Pierce, J. H. et al.,1988). Werden diese Zellen völlig ohne Wachstumsfaktor kultiviert, sterben die Zellen binnen 24 Stunden ab (Apoptose). Dasselbe gilt für Zellen, die in Anwesenheit von EGF und selektiven Inhibitoren des EGF-R inkubiert werden. Die Proliferations- bzw. Apoptoserate ist abhängig von der Konzentration des Inhibitors. Zur Kontrolle für eine unspezifische Toxizität der Testsubstanzen kann der Einfluß der Testsubstanzen auf die IL-3-abhängige Proliferation gemessen werden. Diese Zellen eignen sich zum Screenen nach selektiven Inhibitoren des EGF-R.

Ebenso wie der EGF-Rezeptor wird der HER2-Rezeptor mit der Entstehung epithelialer Tumoren in Verbindung gebracht. Ähnlich wie für die EGF-Rezeptor-Testzelle wurde in den Versuchen der vorliegenden Erfindung eine Testzelle auf der Grundlage der parentalen Zellinie FDC-P1 für den HER2-Rezeptor etabliert. Da der HER2-Rezeptor im Gegensatz zum EGF-Rezeptor normalerweise als Homodimer keine mitogenen Signale transduziert, wurde für die Etablierung der Testzellen eine HER2-Mutante verwendet, bei der die Aminosäure Valin in Position 654 durch die Aminosäure Glutaminsäure ersetzt wurde. Dieser Aminosäureaustausch entspricht der Mutation im neu-Onkogen der Ratte (Weiner, et al., 1989; Suda, et al., 1990) und führt dazu, daß der HER2-Rezeptor auch ohne Liganden und ohne Bildung von Heterodimeren konstitutiv aktiv ist. Ektope Expression des mutierten Rezeptors in FDC-P1 Zellen induziert IL-3 unabhängige Proliferation. Ähnlich wie die EGF-R-Testzelle ist die HER2-Testzelle geeignet, selektive Inhibitoren des Rezeptors zu finden.

Der HGF-Rezeptor (c-met) spielt sowohl bei der Tumorentstehung als auch, bedingt durch das ubiquitäre Vorhandensein seines Liganden HGF im umgebenden Bindegewebe, bei der Tumorinvasion und Metastasierung eine Rolle. Der HGF-Rezeptor ist häufig in Tumorgeweben überexprimiert und/oder amplifiziert. Es wurden bisher keine selektiven Inhibitoren der HGF-R-assoziierten Tyrosinkinase beschrieben. Im Rahmen der vorliegenden Erfindung wurde zur Suche nach Inhibitoren des HGF-R einerseits analog zum EGF-R ein zelluläres Testsystem auf der Grundlage von FDC-P1 Zellen entwickelt, die genetisch so verändert wurden, daß sie den funktionellen humanen HGF-R exprimieren. Diese Zellen benötigen entweder IL-3 oder HGF zum Überleben. Andererseits wurden KB-Zellen, die den humanen HGF-R (und den humanen EGF-R) exprimieren, mit einem Reportergen stabil transfiziert, sodaß Aktivierung des Rezeptors mit HGF (bzw. EGF) eine Induktion des Reportergenprodukts, i.e. der Luciferase, bewirkt, die leicht gemessen werden kann.

Eine wichtige Voraussetzung für das Tumorwachstum ist die Blutversorgung. Diese wird durch die Bildung neuer Blutgefäße im Tumor, Neoangiogenese, gewährleistet. Dazu produzieren Tumorzellen in Sauerstoffnot den Botenstoff VEGF, der nach Bindung an den entsprechenden Rezeptor "KDR", der eine Rezeptor-Tyrosinkinsase (RTK) ist, die Proliferation und Migration der Endothelzellen in Richtung Tumor induziert. Die Blockade der Neoangiogenese durch Inhibition des VEGF-Rezeptors KDR stellt daher einen wichtigen Ansatz in der Tumortherapie dar. Analog zu den Testzellen für EGF-R und HER2 wurden Testzellen für den VEGF-Rezeptor KDR hergestellt. Expression und Aktivierung dieser RTK führt zur IL-3-unabhängigen Proliferation und eignet sich daher zum Screenen nach selektiven Inhibitoren.

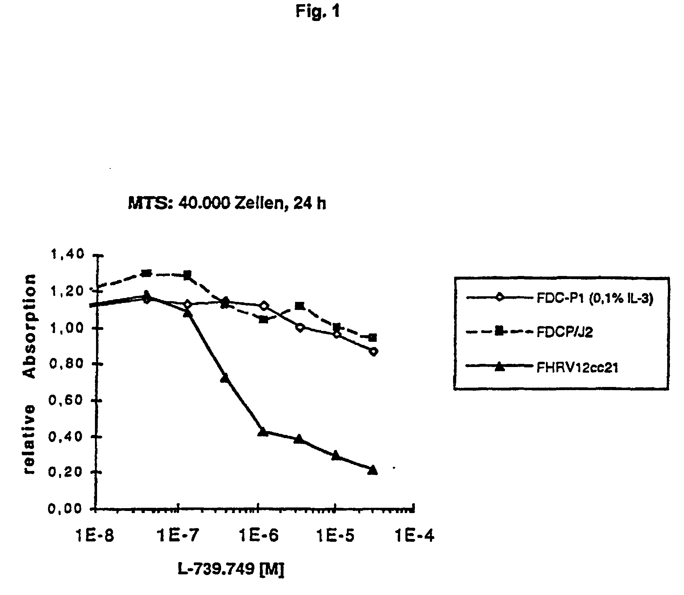

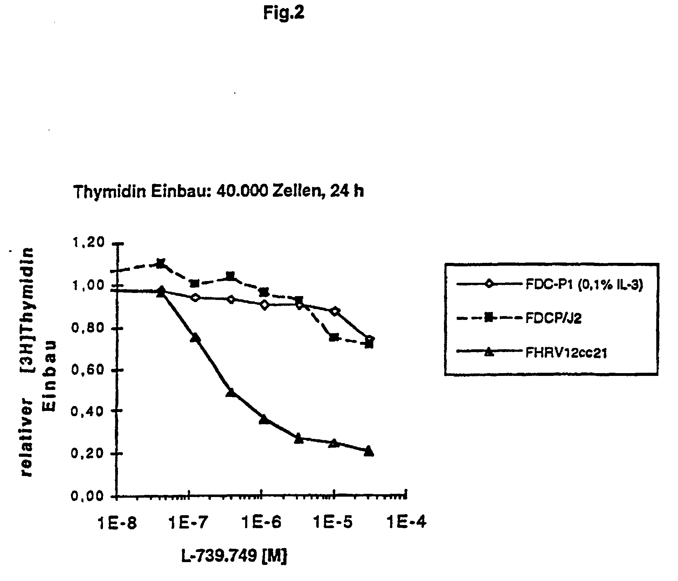

Mutationen der Ras-Proteine H-Ras, K-Ras und N-Ras, die zu einer konstitutiven Aktivierung von Ras führen, werden in vielen menschlichen Tumoren gefunden (Bos, 1988; Kiaris und Spandidos, 1995). Das Auftreten von Ras-Mutationen gilt als wichtiger Schritt in der Tumorentstehung, daher bieten sich Ras-Mutanten als Zielmoleküle bei der Tumorbehandlung an. Verschiedene Ras-Inhibitoren, wie monoklonale Antikörper (Furth et al., 1982), dominant-negative Mutanten (Stacey et al., 1991; Quilliam et al., 1994), Antisense-RNA oder Inhibitoren der Ras-Farnesyltransferasen (Kohl et al., 1993; Kohl et al., 1994; Kohl et al., 1995), haben die Richtigkeit des therapeutischen Prinzips, Ras-Mutanten als Interventionsstellen bei der. Tumorbehandlung heranzuziehen, bestätigt. Die derzeit verfügbaren Inhibitoren eignen sich jedoch nur beschränkt für therapeutische Zwecke, da Ras-spezifische Inhibitoren (Antikörper, dominant-negative Mutanten oder Antisense-RNA) unter *in vivo* Bedingungen nicht in ausreichenden Konzentrationen in alle Tumorzellen eingebracht werden können, und andererseits die Farnesyltransferase-Inhibitoren nicht selektiv für mutiertes Ras sind, sondern auch die posttranslationale Modifikation von normalem Ras, sowie anderer zellulärer Proteine, wie z.B. Lamin A und B, Skelettmuskel-Phosphorylasekinase, und Retinaproteine wie Transducin und Rhodopsinkinase beeinflussen. Ferner ist K-Ras, welches in humanen Tumoren am häufigsten mutiert ist, auch Substrat für die posttranslationale Modifikation durch Geranylgeranyl-Transferase, welche durch die Farnesyltransferase Inhibitoren nur unzureichend blockiert wird (James et al., 1995; Lerner et al., 1995; Manne et al., 1995). Mit den im Rahmen der vorliegenden Erfindung entwickelten Testzellen können niedermolekulare Inhibitoren der aktivierten Ras-Proteine gesucht werden. Im Rahmen der vorliegenden Erfindung wurden als Testzellen für potentielle Ras-Inhibitoren ebenfalls FDC-P1 Zellen verwendet. Ektopische Expression der aktivierten Ras-Mutanten K-Ras^{Val12} (McCoy et al., 1984) und H-Ras^{Val12} (Reddy et al., 1982) induziert IL-3-unabhängige Proliferation der Testzellen (Fig. 1 und 2). Die Verwendung eines literaturbekannten Ras-Inhibitors, z.B. des Farnesyltransferaseinhibitors L-739.749 (2(*S*)-{2(*S*)-[2(*R*)-Amino-3-mercapto]propylamino-3(*S*)-methyl}pentyloxy-3-phenylpropionylmethioninsulfonmethyl ester; Kohl et al., 1994)) zeigte die Abhängigkeit der Testzellen von der Anwesenheit des aktivierten Ras-Proteins (Fig. 1 und 2). Die im Rahmen der vorliegenden Erfindung verwendeten Zellen eignen sich zur Suche nach niedermolekularen Ras-Inhibitoren und können in einem High Throughput-Screen eingesetzt werden.

Zur Überprüfung der Spezifität von potentiellen Ras-Inhibitoren können Kontrollzellen verwendet werden, die durch die Expression aktivierter Proteine IL-3-unabhängig gemacht wurden, die im Ras-Signaltransduktionsweg Ras nachgeschaltet sind und damit das Zellwachstum unabhängig von Ras machen (z.B. der über Raf, MEK und ERK (= MAP Kinase) oder der über Rac und Rho laufende Signalübertragungsweg - gegebenenfalls in Kombination mit einem zweiten Signal wie z.B. c-myc). Eine Zellinie dieser Art ist FDC-P1/J2, die durch Infektion mit einem Retrovirus, welches die Expression von aktiviertem v-raf und v-myc bewirkt, hergestellt wurde (Rapp et al., 1985). Neben der Wirkung der genannten Zielmoleküle gilt die Überexpression des Bcl-2 Proteins als eine der Ursachen für bestimmte maligne Erkrankungen, wie z.B. Leukämien (Reed, 1994), außerdem wurde eine Überexpression dieses Proteins auch in Tumoren epithelialen Ursprungs beschrieben (McDonnell, et al., 1992; Pezzella, et al., 1993; Lu, et al., 1993). Es gibt einen klaren Zusammenhang zwischen Bcl-2 Spiegel und Resistenz gegen Chemotherapeutika (Kitada, et al., 1994; Miyashita und Reed, 1992). Der molekulare Wirkmechanismus von Bcl-2 ist die Verhinderung des programmierten Zelltodes (Reed, 1994). Bcl-2 gilt somit als potentiell wichtiges Angriffsziel in der Therapie bestimmter Leukämien und solider Tumore. Zum Screenen nach niedermolekularen Inhibitoren des Bcl-2-Proteins können z.B. FDC-P1-Testzellen, die Bcl-2 überexprimieren, verwendet werden; diese Zellen überleben auch in Abwesenheit von IL-3.

Mit Hilfe eines Screens auf der Grundlage dieser Zielmoleküle erhaltene Substanztreffer können in einem einzigen Arbeitschritt, und zwar bereits im Primärscreen, differentiell nach ihrer Wirkung auf die Zielmoleküle, die aufgrund unterschiedlicher Mechanismen am Tumorgeschehen beteiligt sind, klassifiziert werden. Damit ist es möglich, Substanzen mit sehr spezifischer Wirkung für die Therapie bestimmter Tumoren zu finden.

Außer den im Ausführungsbeispiel 1 der vorliegenden Erfindung, in dem nach potentiellen Tumortherapeutika gescreent wurde, verwendeten Zellen der Bezeichnung PDC-P1, deren Herstellung von Dexter, T. M. et al., 1980, beschrieben wurde, können für Screens auf der Grundlage der Messung der Proliferation als Nachweissystem andere Wachstumsfaktor-abhängige Zellen verwendet werden; z.B. die von Pierce, J. H. et al., 1988, beschriebene Zellinie der Bezeichnung 32D, oder die von Shibuya, H. et al., 1992, bzw. Alexander, W. S. et al., 1991, beschriebenen Zellinien der Bezeichnung BAF-B03 bzw. DA-1.

Das Assay-System kann z.B. wie folgt aufgebaut werden: In 96-Well Mikrotiterplatten werden die Zellen in Gegenwart des entsprechenden Wachstumsfaktors vorgelegt. Anschließend gibt man, z.B. mit Hilfe eines Pipettierroboters wie Tecan RSP5052, Verdünnungen der zu testenden Substanzen zu und inkubiert die Testzellen für einen bestimmten Zeitraum unter zellkulturüblichen Bedingungen. Anschließend werden die Zellen mit Assayreagenz, z.B. dem auf MTS basierenden "Cell titer non-radioactive proliferation assay" (Promega) versetzt und nach einer weiteren kurzen Inkubation in einem Mikrotiterplattenphotometer (z.B. SLT Spectra Shell) gemessen. Dabei muß die Inkubationszeit so bemessen sein, daß Testzellen, denen der Wachstumsfaktor entzogen wurde, und Testzellen, die in Gegenwart von Wachstumsfaktor in diesem Zeitraum einen deutlichen Unterschied in der Zellzahl aufweisen. Substanzen, die spezifisch eine Rezeptortyrosinkinase (RTK) inhibieren, dürfen in Zellinien, die von einer anderen RTK abhängig sind, keinen Effekt haben.

Grundlage eines weiteren im Rahmen der vorliegenden Erfindung eingesetzten Assays ist die humane epitheliale Zellinie KB4, in die das Luciferasegen, unter der Kontrolle des humanen fos-Promotors, eingebracht wurde. Diese Zellen exprimieren endogen den humanen EGF-Rezeptor, den humanen HGF-Rezeptor sowie einige weitere Rezeptortyrosinkinasen. Behandlung der Zellen nach Serumentzug mit einem Liganden dieser Rezeptortyrosinkinasen führt zu Aktivierung des fos-Promotors und zur Expression des Luciferasegens, dessen Aktivität über einen gewissen Bereich proportional zur Stärke des Aktivierungssignals ist. Ein entsprechendes Versuchssystems für ein High Throughput Screening kann folgendermaßen aufgebaut werden: In 96-Well Mikrotiterplatten werden die oben beschriebenen Zellen in serumreduziertem Medium vorgelegt. Anschließend werden mit Hilfe eines Pipettierroboters (z.B. Tecan RSP5052) Verdünnungen der zu testenden Substanzen zugegeben und das unter fos-Kontrolle stehende Luciferasegen mit dem entsprechenden Wachtumsfaktor induziert. Die Testzellen werden für einen bestimmten Zeitraum unter zellkulturüblichen Bedingungen inkubiert. Anschließend werden die Zellen mit einer Pufferlösung gewaschen und mit einem Assayreagenz versetzt, das die Zellen lysiert und die für die Luciferasereaktion nötigen Komponenten Luciferin und ATP enthält. Nach einer kurzen Inkubation wird das Luciferasesignal in einem Mikrotiterplattenluminometer (SLT Spectra Shell; Wallac Microbeta) gemessen. Ein spezifischer RTK Inhibitor reduziert das Luciferasesignal in der entsprechenden Testzelle, hat aber auf die c-fos-Promotoraktivität in anderen Testzellen keinen Einfluß.

### Figurenübersicht

- Fig.1:: Hemmung der H-ras^{Val12} abhängigen Proliferation (Viabilität) durch den Farnesyltransferase-Inhibitor L-739.749 in IL-3 freiem Medium
- Fig.2:: Hemmung der H-ras^{Val12} abhängigen Proliferation (DNA-Synthese) durch Farnesyltransferase-Inhibitor L-739.749 in IL-3 freiem Medium
- Fig.3:: Nachweis der Überexpression von H-ras^{Val12} in FHRV12cc21 Zellen und Hemmung der Ras-Farnesylierung durch L-739.749
- Fig.4:: Nachweis der HGF-abhängigen Proliferation bzw. Viabilität durch Messung des ³H-Thymidin Einbaus bzw. der Absorption für verschiedene Konzentrationen von rekombinantem HGF
- Fig.5:: Dosisabhängige Induktion der Luciferaseaktivität in KB-B4 Zellen durch HGF bzw. EGF
- Fig.6:: Selektive Hemmung der EGF-induzierten, nicht jedoch der HGF-induzierten Luciferaseaktivität durch einen EGF-R-Inhibitor
- Fig.7:: Selektive Hemmung der Proliferation einer EGF-abhängigen Zellinie durch einen EGF-R-Inhibitor

### Beispiel 1

### Proliferationsassay im High Throughput-Format zum Screenen von Substanzen mit anti-Tumorwirkung

### a) Herstellung einer Testzelle für aktiviertes H-Ras^{Val12}

### i) Expressionsvektor für humanes H-Ras^{Val12}

Als Vektor für die ektopische Expression von aktivierten humanen Ras-Proteinen eignen sich retrovirale Vektoren wie z.B. pGD (Daley et al., 1990), pLXSN (Miller und Rosman, 1989)., oder Expressionsplasmide mit starken eukaryotischen oder viralen Promotoren wie z.B. SV40 oder Cytomegalovirus (CMV), unter deren transkriptionellen Kontrolle die cDNA oder das Gen für ein aktiviertes Ras-Protein gestellt werden. Für die Herstellung der Testzelle wurde das Expressionsvektor pGDV12ras (Tanaka et al., 1994) verwendet, das durch Klonierung der onkogenen Form der humanen cDNA für c-Hras^{Val12} in die EcoRI-Stelle des retroviralen Vektors pGD (Daley et al., 1990) unter die Kontrolle des murinen retroviralen LTR gebracht wurde. Der pGD Vektor enthält zusätzlich das Neomycin-Phosphotransferase-Gen unter der Kontrolle eines Thymidinkinase Promotors aus Plasmid pMC1neo (Stratagene), welches mit Neomycin-Analogen (G-418) die Selektion von Zellen ermöglicht, die das Expressionskonstrukt stabil in ihr Genom integriert haben.

### ii) Transfektion

FDC-P1 Zellen (Dexter et al., 1980) wurden in IMDM Medium (BioWhittaker), versetzt mit 10% hitzeinaktiviertem fötalem Kälberserum (FCS), 2 mM Glutamin, 50 µM β-Mercaptoäthanol und 1% IL-3 angesetzt und bei 37°C in 5% CO₂ inkubiert. 10⁷ Zellen pro Transfektion wurden 5 min bei 1200 UpM bei Raumtemperatur abzentrifugiert (Heraeus Minifuge), einmal mit serumfreiem RPMI-1640 Medium gewaschen, und in 1 ml serumfreiem Medium suspendiert. 800 µl dieser Zellsuspension wurden in einer Elektroporationsküvette mit 20 µg Plasmid pGDV12ras gemischt und mit einem einzelnen Stromstoß von 280 V, 980 µF und 1000 ms elektroporiert (Progenetor II, Hoefer Instruments). Die Zellen wurden in 10 ml Kulturmedium mit 1% IL-3 verdünnt und bei 37°C, 5% CO₂ inkubiert.
Zwei Tage nach der Transfektion wurden die Zellen mit frischem Medium 3-fach verdünnt und 250 µg/ml G-418 zur Selektion auf Neomycin-resistenz zugesetzt. An den darauffolgenden Tagen wurde die G-418 Konzentration auf 500, 750 und 1000 µg/ml erhöht. Tote Zellen wurden mittels Zentrifugation durch einen Ficoll-Gradienten entfernt. Dazu wurden die Zellen zuerst mittels Zentrifugation (5 min, 1200 UpM) in einem geringen Volumen aufkonzentriert und in einem Zentrifugenröhrchen über Ficoll-Paque (Pharmacia) geschichtet. Nach Zentrifugation (15 min, 2500 UpM, 20°C) und freiem Auslauf der Zentrifuge wurden die an der Medium/Ficoll-Zwischenphase angesammelten lebenden Zellen abpipettiert, einmal mit Medium gewaschen und in frisches Medium überführt.
Acht Tage nach Beginn der G-418 Selektion wurde begonnen, IL-3 unabhängige Zellen anzureichern, indem die IL-3 Konzentration schrittweise verringert wurde. Nachdem die Zellen eine Woche lang in 0.0005% IL-3 kultiviert waren, wurden sie durch Aussaat in halbflüssigem Methocel-Medium vereinzelt. Entstandene, von anderen Zellen gut getrennte Zellkolonien wurden isoliert, in 96-Loch Platten mit 100 µl Medium (ohne IL-3) überführt und in weiterer Folge expandiert. Eine der erhaltenen IL-3 unabhängigen stabilen Zellinien, die gute Wachstumseingenschaften aufwies, wurde FHRV12cc21 benannt.

### iii) Charakterisierung der Zellen

### Nachweis der H-ras^{Val12} Expression

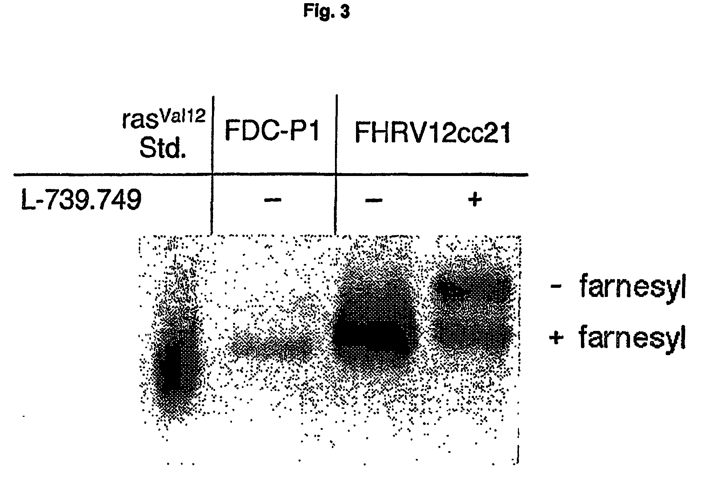

FHRV12cc21 und untransfizierte FDC-P1 Zellen wurden in IMDM Medium mit 10% FCS und 1% IL-3 gezüchtet. Zum Nachweis der Hemmung der posttranslationalen Modifikation von H-ras^{Val12} durch Farnesylierung wurden die Zellen für 48 Stunden mit 5 µM des Protein-Farnesyltransferase-Inhibitors L-739.749 (Kohl et al., 1994) inkubiert. Die Zellen wurden mit PBS gewaschen und in PBSTDS (1% Triton X-100, 0.5% Natriumdesoxycholat, 0.1% Natriumdodecylsulfat in PBS) 5 min bei 4°C lysiert. Die Zellysate wurden durch Zentrifugation (20 min 14.000x *g*, 4°C) geklärt. Ras Proteine wurden durch Zusatz von 1 µg monoklonalen Ratten Antikörper Y13-238 (Oncogene Science) und Protein-G Plus / Protein-A Agarose (Oncogene Science) über Nacht bei 4°C immunpräzipitiert. Die Präzipitate wurden zweimal mit PBSTDS Puffer gewaschen und anschließend in SDS-Probenpuffer mit 3% β-Mercaptoethanol ausgenommen. Die Proteine wurden in einem 20%igen SDS-Polyacrylamidgel aufgetrennt, auf eine Immobilon-Membran (Millipore) transferiert und nach Absättigen der Membran mit 10% FCS, 1% Rinderserumalbumin, 1% Tween-20 in PBS, durch aufeinanderfolgende Inkubation mit einem Ras^{Val12} spezifischen monoklonalen Antikörper (pan-Ras^{Val12}, Oncogene Science) und einem sekundären anti-Maus-IgG - Alkalische Phosphatase-Konjugat durch Färbereaktion mit NBT und BCIP (Promega) nachgewiesen. Es wurde eine deutliche Überexpression von H-Ras^{Val12} in FHRV12cc21 Zellen gegenüber der untransfizierten FDC-P1-Kontrollzelle und die Hemmung der Ras-Farnesylierung durch L-739.749 gezeigt. Die mit L-739.749 behandelten Zellen enthielten mehr als 50% des Ras-Proteins in der unmodifizierten (nicht farnesylierten), und deshalb im SDS-Gel langsamer wandernden Form. (Der Nachweis der Überexpression von H-ras^{Val12} in FHRV12cc21 Zellen und die Hemmung der Ras-Farnesylierung durch L-739.749 sind in Fig. 3 dargestellt. Nach Behandlung mit (+) oder ohne (-) 5 µM L-739.749 für 48 h in Gegenwart von 1% IL-3 wurde Ras Protein aus Zellysaten mit anti-Ras Antikörper immunpräzipitiert und nach elektrophoretischer Auftrennung in einem SDS-Polyacrylamidgel und Membrantransfer mit anti-Ras^{Val12} Antikörper nachgewiesen. Mit "ras^{Val12} Std." ist eine K-Ras^{Val12} Standardpräparation (Oncogene Science) bezeichnet). Nach Behandlung mit (+) oder ohne (-) L-739.749 für 48 h in Gegenwart von 1% IL-3 wurde Ras-Protein aus Zellysaten mit anti-Ras-Antikörper immunpräzipitiert und nach elektrophoretischer Auftrennung in einem SDS-Polyacrylamidgel und Membrantransfer mit anti-Ras^{Val12} Antikörper nachgewiesen. Mit ras^{Val12} Std. ist eine K-Ras^{Val12} Standardpräparation (Oncogene Science) bezeichnet.

### iv) Hemmung des IL-3 unabhängigen Wachstums von H-ras^{Val12} transfizierten Zellen durch Hemmung der Ras Farnesylierung

40.000 Zellen wurden in 100 µl RPMI-1640 Medium ohne Phenolrot (BioWhittaker), supplementiert mit 10% FCS, 2% Glutamin, und 50 µM β-Mercaptoäthanol, in 96-Loch Platten ausgesät. Untransfizierte FDC-P1 Zellen wurden im gleichen Medium unter Zusatz von 0.1% IL-3 enthaltendem Zellüberstand kultiviert, damit die Zellen nicht in die Apoptose getrieben wurden. Eine Stammlösung des Farnesyltransferase-Inhibitors L-739.749 (10 mM in DMSO) wurde mit PBS unter Zusatz von DMSO seriell verdünnt und 10 µl der Verdünnungen wurden zu den Zellen pipettiert und anschließend bei 37°C und 5% CO₂ inkubiert. Die endgültige DMSO-Konzentration betrug in allen Fällen 0.5% (v/v). Die Anzahl der lebenden Zellen wurde indirekt durch einen photometrischen MTS-Assay (siehe oben) bestimmt. Für die Bestimmung der DNA-Syntheserate wurden 16 h vor der Messung 10 µl [6-³H]Thymidin in PBS (0.1 µCi; 27 Ci/mmol, Amersham) pro Napf zugegeben. Die Zellen wurden mittels eines Zellerntegeräts (Canberra Packard) auf Filterplatten übertragen, mit Wasser gewaschen und die in die DNA eingebaute [³H]Thymidinmenge in einem Szintillationszähler (TopCount, Canberra Packard) ermittelt. Die in Fig. 1 und Fig. 2 dargestellten Werte entsprechen dem Mittelwert von Triplikat-Bestimmungen, bezogen auf die unbehandelten Zellen. (In den Fig. 1 und 2 ist die Hemmung der H-ras^{Val12} abhängigen Proliferation (Viabilität, angegeben als relative Absorption, in Fig. 1; DNA-Synthese in Fig. 2) durch Farnesyltransferase-Inhibitor L-739.749 in IL-3 freiem Medium dargestellt. FDC-P1: IL-3-abhängige untransfizierte Ausgangszellen.

FDC-P1/J2: IL-3 und Ras-unabhängige, mit aktiviertem v-myc plus v-raf transformierte FDC-P1 Zellinie. FHRV12cc21: IL-3 unabhängige, mit humanem H-ras^{Val12} transformierte FDC-P1 Zellinie..)

### b) Herstellung von KDR-Testzellen

### i) Klonierung von KDR und Herstellung von Expressionsplasmiden (Rezeptor-DNA)

Die cDNA des humanen KDR wurde durch Screenen einer cDNA-Bank erhalten und in die Expressionsvektoren pRc/CMV (Invitrogen V750-20) bzw. LXSN (Miller, et al., 1989) hineinkloniert.

### ii) Isolierung von Klonen, die die für den humanen KDR kodierende Sequenz enthalten

Durch Screenen einer humanen Endothelzellen-cDNA Bank im Lambda gt11 Vektor (Clontech HL 1024b) mit PCR-Sonden, erhalten durch "nested" Amplifizierung zweier KDR-Genfragmente (Terman et al., 1992; EMBL Accession Nr. X61656) unter Verwendung von aus derselben Bank extrahierten DNA als Vorlage, wurden 13 Klone isoliert und die Inserts, enthalten im Plasmid pUC18, sequenziert. Die für die PCR-Amplifizierung verwendeten Sense-Primer entsprachen den Aminosäure-Sequenzen RGQRDLDWLWP, WDSKKGFTIP , GARFRQGKDYVG, und CWHGEPSQRP, Positionen 52-62, 177-186, 940-951 bzw. 1140-1149. Die Antisense-Primer entsprachen den Aminosäure-Sequenzen TLVIQAANVSA und SGYHSDDTDTT, Positionen 514-524 bzw. 1305-1315. Der Full-Length cDNA-Klon wurde durch Ligation von drei überlappenden Klonen und zwei Paaren komplementärer Oligonukleotide, kodierend für die 38 Aminosäuren der publizierten Sequenz, die nicht bis zum Start-ATG geht, plus vier zusätzliche Aminosäuren, einschließlich das erforderliche ATG, zusammengebaut. Nukleotide, die die effiziente Translation des Proteins begünstigen, wurden stromaufwärts vom ATG eingefügt (GGATCCCTCGACGCGCC; die BamHI-Stelle am Beginn dieser Sequenz dient Klonierungszwecken). Die Ligierung der synthetischen Oligonuklotide wurde durch die Schaffung einer EcoRV-Stelle bei den Aminosäuren 37-38 (GAC ATA wurde zu GAT ATC mutiert, ohne die Sequenz für die Aminosäuren DI zu verändern) möglich. Die Sequenzen der erhaltenen cDNA-Klone unterscheiden sich von der publizierten Sequenz (Terman et al., 1992; EMBL-Datenbank, Accession Nr. X61656) an den Positionen 770 (Ala statt Thr), 785 (Arg statt Gly), 846 (Val statt Glu), und 1345 (Ser statt Thr). Diese Änderungen machen die erhaltene Sequenz an den entsprechenden Postitionen identisch mit Maushomologen von KDR, Flk-1 (Matthews et al. 1991; GenBank Accession Nr. X59397; Millauer et al., 1993; EMBL Accession Nr. X70842), außer an der letzten Position, die in einem Bereich geringer Konservierung liegt. Außerdem liegen zwei der betroffenen Aminosäuren, nämlich 770 und 785, in dem Bereich, von dem berichtet wurde, daß er die Transmembrandomäne darstellt (Aminosäuren 763 bis 787, Terman et al., 1992). Dadurch wurde es erforderlich, die Randbereiche wieder an die Reste 763 bis 784, entsprechend Flk-1 (Matthews et al., 1991), anzugleichen.

### iii) Subklonierung der KDR-cDNA in den Expressionsvektor pRc/CMV

Die vollständig zusammengesetzte KDR-cDNA wurde zuerst mit Acc65 (isoschizomer KpnI) und SalI aus pUC18-KDR herausgeschnitten und in die Acc65- und SalI-Stelle von pAD-CMV2 (EP-A 393 438) subkloniert. Dann wurde das erhaltene Plasmid pAD-CMV2-KDR mit XhoI geschnitten, die DNA-Enden unter Verwendung des Klenow-Fragments von DNA-Polymerase I in Gegenwart aller vier dNTPs aufgefüllt, Phenol-extrahiert und mit Ethanol ausgefällt. Die erhaltene stumpfendige ("blunt end") DNA wurde mit XbaI nachverdaut. Der Expressionsvektor pRc/CMV wurde mit HindIII geschnitten, die DNA-Enden "blunt end" gemacht, und der Vektor danach mit XbaI geschnitten. In diesen Vektor wurde die aus dem Plasmid pAD-CMV2 herausgeschnittene KDR-Sequenz, die ein stumpfes und ein XbaI-Ende aufweist, hineinligiert, das dadurch entstandene Plasmid wurde pRc/CMV-KDR genannt.

### iv) Klonierung der KDR-Sequenz in den Expressionsvektor pLXSN

Der Expressionsvektor pLXSN wurde mit Hpa I geschnitten. In diesen Vektor wurde die mit XhoI und XbaI doppelt aus dem Plasmid pAD-CMV2-KDR herausgeschnittene und danach durch Zugabe aller vier dNTPs mittels Klenow-Enzym "blunt end" gemachte KDR-Sequenz hineinligiert. Ein so erhaltener Klon mit der richtigen Orientierung wurde pLXSN-KDR genannt.

### v) Transfektion von FDC-P1 Zellen

1 x 10⁷ FDC-P1 Zellen wurden mit 1200 rpm bei Raumtemperatur abzentrifugiert, einmal mit serumfreien RPMI gewaschen und in 0.8 ml serumfreien RPMI resuspendiert. Es wurden 20 µg pRc/CMV-KDR, linearisiert mit ScaI, zugesetzt und bei 280 V, 980 Mikrofarad, 1 sec elektroporiert. Elektroporierte Zellen wurden in Iscoves Modified Dulbecco's Medium (IMDM) mit Interleukin-3 (IL-3) und 10% fötalem Kälberserum (FCS) resuspendiert und in Kultur gehalten. Zwei Tage nach der Transfektion wurde die Selektion von Transfektanten durch Zugabe von 400 µg/ml Geneticin (G418) gestartet. Weitere zwei Tage später wurde die G418-Konzentration auf 800 µg/ml und in der Folge auf 1000 µg/ml gesteigert. Durch G418 abgestorbene Zellen wurden von resistenten Zellen durch Zentrifugation in einem Ficoll-Gradienten abgetrennt. Dazu wurde die Mischung aus toten und lebenden Zellen abzentrifugiert, wie oben beschrieben, in 1 ml IMDM + IL-3 + 10% FCS + 1 g/l G418 resuspendiert und mit 1 ml Ficoll (Pharmacia) unterschichtet. Der Gradient wurde bei Raumtemperatur bei einer Geschwindigkeit von 2500 rpm 15 min gefahren. In der Zwischenphase befinden sich die lebenden Zellen, während tote Zellen pellettiert werden. Die Zellen der Zwischenphase werden zweimal mit je 10 ml IMDM + IL-3 + 10% FCS + 1 g/l G418 gewaschen und in demselben Medium weiter kultiviert.

Um G418 resistente Zellen zu erhalten, die unabhängig von IL-3, aber in Abhängigkeit von VEGF wachsen, wurde die IL-3 Konzentration auf ein Zehntel reduziert und gleichzeitig 10 ng/ml humanes rekombinantes VEGF 165 (Fiebich et al., 1993) zugegeben. Abgestorbene Zellen wurden von den lebenden wiederum durch Ficoll-Gradienten abgetrennt. Die Reduktion von IL-3 erfolgte jeweils auf ein Zehntel der vorangegangenen IL-3 Konzentration, bis die Zellen unabhängig von IL-3 gehalten werden konnten.

Um Einzelklone zu erhalten, wurden diese Zellen in Methocel auskloniert (Metcalf et al., 1992; Meckling-Gill et al., 1992). Die Ausklonierung erfolgte in Abwesenheit von G418, aber in der Anwesenheit von VEGF.

Die Zell-Klone (FDCP-CMV-KDR) wurden auf ihre Stimulierbarkeit durch VEGF getestet. Dazu wurden die Zellen in 96-Loch Platten ausgesät (15000 Zellen/well) und mit oder ohne VEGF kultiviert. Nach 48 Stunden wurde ein MTS-Assay (Promega Non radioactive cell proliferation assay: Cell Titer 96AQ) durchgeführt und das Zellwachstum mittels dieses kolorimetrischen Assays bestimmt. Keiner der Klone zeigte verstärktes Wachstum in Anwesenheit von VEGF. Wie mittels RT-PCR (Reverse-Transkriptase Polymerase-Kettenreaktion) gezeigt werden konnte, produzieren FDC-P1 Zellen und die transfizierten Zellen (FDCP-CMV-KDR) selbst VEGF. FDCP-CMV-KDR Zellen stimulieren sich somit autokrin.

### vi) Infektion von FDC-P1 Zellen

GP+E-86, eine Verpackungszellinie für Retroviren (Markowitz et al., 1988.), wurde in 60 mm Kulturschalen in IMDM mit 10% FCS zu 70-80% Konfluenz wachsen gelassen. Diese Zellen wurden mittels Lipofectamine Reagens (Gibco BRL) nach Vorschrift des Herstellers mit 5 µg nicht geschnittener (supercoiled) DNA (pLXSN-KDR) und 10 µl Lipofectamine über Nacht transfiziert. Am nächsten Tag wurden die Zellen mit Trypsin abgelöst, abzentrifugiert, in Medium aufgenommen, in Kulturflaschen (75 cm²) überführt und die Selektion von Transfektanten durch Zugabe von 800 µg G418/ml gestartet. Nach weiteren zwei Tagen wurde die G418 Konzentration auf 1000 µg/ml erhöht. Nach der Selektion wurden die GP+E-86-LXSN-KDR Zellen mit 10 ng/ml VEGF gehalten. Um FDC-P1 Zellen zu infizieren, wurden die resistenten GP+E-86-LXSN-KDR Zellen gepoolt und 500.000 Zellen in 60 mm Kulturschälchen in IMDM mit 10% FCS ohne G418 ausgesät. 100.000 - 200.000 FDC-P1 Zellen wurden zugegeben und in Anwesenheit von 1% IL-3 für 48 Stunden kokultiviert. Die infizierten FDC-P1 Zellen (FDCP-LXSN-KDR) wurden in Kulturflaschen überführt und in Anwesenheit von VEGF und 1% IL-3 gehalten. Zwei Tage nach Co-Kultur wurde mit der Selektion in G418 begonnen. Die Selektion, IL-3 Reduktion und Ausklonierung erfolgte, wie unter v) für die Transfektion von Zellen (FDCP-CMV-KDR) beschrieben.

### c) Herstellung von EGF-R(Epidermal Growth Factor Receptor)-Testzellen

EGF-R-Testzellen der Bezeichnung F/LHERc Zellen wurden aus der Interleukin-3-abhängigen hämatopoetische Mauszellinie FDC-P₁ durch Infektion mit einem rekombinanten, die für den menschlichen EGF-Rkodierende DNA enthaltenden Retrovirus hergestellt. Das zur Expression der humanen EGF-R-cDNA (Ullrich et al., 1984) verwendete rekombinante Retrovirus entspricht dem in von Rüden, T. et al., 1988, beschriebenen Konstrukt mit dem Unterschied, daß zur Expression der EGF-R cDNA (wie in a) zur Verwendung von KDR) der retrovirale Vektor LXSN (Miller al., 1989) eingesetzt wurde und als Verpackungszelle die Linie GP+E86 (Markowitz al. 1988) diente. Selektion und Klonierung der Zellen wurden im wesentlichen vorgenommen, wie in a) beschrieben. Zur Selektion (Virus-infizierter FDC-P1-Zellen wurden die Kulturen in Anwesenheit von 1% IL-3 (1% konditioniertes Medium von der IL-3-sezernierenden Zellinie X63-0 mIL-3; Karasuyama und Melchers, 1988), um maximale IL-3-Stimulation sicherzustellen, in 1000 µg/ml G418 ausselektiert, da das Viruskonstrukt neben der humanen EGF-R cDNA auch ein Neomycin-Resistenzgen trägt. Anschließend wurde den Kulturen G418-resistenter Zellen 20ng/ml humanes rekombinantes EGF (Promega) zugesetzt und die IL-3-Konzentration sukzessive von 1% auf Null zurückgeführt. Zellen, die in Abwesenheit von IL-3 EGFabhängig wuchsen, wurden schließlich in Methylcellulose in Anwesenheit von TGF-α, aber nicht IL-3 kloniert (wie EGF stimuliert auch TGF-α den EGF-Rezeptor, ist aber stabiler). Abschließend wurde von jedem isolierten Klon bestätigt, daß die Zellen in Abwesenheit von EGF und IL-3 sterben, daß sie in Gegenwart von EGF oder IL-3 als einzigem Wachstumsfaktor proliferieren, und daß die EGF-Stimulierung durch bekannte EGF-Rezeptor-Inhibitoren aufgehoben wird.

### d) Herstellung von HER2-Testzellen

### i) Subklonierung der mutanten HER2-Sequenz in den Expressionsvektor pAHygCMV1

Aus Plasmid pHer2-CVN, das die komplette 4.5 kb große kodierende Sequenz des humanen Rezeptors HER2 enthält (GeneBank AC-Nr. M11730; Coussens et al., 1985), wurde die HER2-CDNA in 2 Teilen herausgeschnitten und subkloniert: Zuerst wurde das 3.2 kb große SalI-KpnI Fragment, das den 5'-Teil der HER2-cDNA enthält, herausgeschnitten und in den mit SalI und KpnI doppelt geschnittenen Expressionsvektor pAHygCMV1 gerichtet kloniert, sodaß die Transkription von HER2 unter der Kontrolle des Cytomegalovirus (CMV) Promotor/Enhancer Elements steht. Das entstandene Zwischenprodukt wurde zuerst mit NotI geschnitten, die Enden durch Zugabe *von* dNTPs und dem Klenow-Fragment der DNA-Polymerase stumpf gemacht ("blunt ended"), und dann mit KpnI geschnitten. Danach wurde das 0.8 kb große KpnI-StuI Fragment, das den 3'-Teil der HER2-cDNA enthält, aus pHer2-CVN herausgeschnitten und in das Zwischenkonstrukt gerichtet kloniert. Der entstandene Expressionsvektor pAHygCMVHer2 enthält die komplette kodierende Region von HER2 unter der Kontrolle des CMV-Promotors.

Zur Einführung der Punktmutation Val659->Glu659 (GTT->GAA) (Suda et al., 1990) wurde das 3.2 kb große SalI-KpnI HER2-Fragment zuerst in das Plasmid pBluescript KS (Stratagene), das zuvor mit SalI und KpnI geöffnet worden war, kloniert. Zur Mutagenese wurden ein Oligonukleotid, das die AatII Schnittstelle und 16 bp downstream die mutierte Sequenz GAA enthält, und ein Antisense-Oligonukleotid, das die Sequenzen um die NdeI Schnittstelle enthält, synthetisiert. Unter Verwendung dieser beiden Oligonukleotide als Primer wurde mittels PCR (Polymerase Chain Reaction) aus dem SalI-KpnI HER2-Fragment das 400 bp große AatII-NdeI Fragment amplifiziert, das nun die Mutation GTT->GAA enthält. Das amplifizierte Fragment wurde mit AatII und NdeI nachgeschnitten und in das ebenfalls mit AatII und NdeI geschnittene Plasmid pBluescript/Her2(SalI-KpnI) kloniert. Der Bereich von AatII bis NdeI wurde sequenziert um zu gewährleisten, daß außer der gewünschten keine weiteren Mutationen durch die PCR-Amplifikation erhalten wurden. Plasmid pBluescript/Her2(V659E) enthält nun die mutierte Form des 3.2 kb großen SalI-KpnI HER2-Fragments. Dieses mutierte Fragment wurde aus dem Plasmid mit SalI und KpnI wieder herausgeschnitten, das nicht-mutierte Fragment wurde aus dem Expressionsvektor pAHygCMVHer2 ebenfalls mit SalI und KpnI herausgeschnitten, und durch das mutierte Fragment ersetzt. Der resultierende Expressionsvektor, genannt pAHygCMVHer2(V659E), enthält nun die komplette kodierende Region der mutierten, konstitutiv aktiven Form HER2(V659E).

### ii) Transfektion der FDCP-Zellen

FDC-P1-Zelle (Dexter et al., 1980), die IL-3-abhängig wachsen, wurden mit Plasmid pAHygCMVHer2(V659E) folgendermaßen transfiziert: Etwa 1x10⁷ Zellen wurden in IMDM-Medium (Bio-Whittaker) mit 10 µg Plasmid DNA gemischt und mit einem Elektroporator (Hoefer, Progenetor II) bei 980 µF, 280 V für 1000 ms elektroporiert. Die Zellen wurden in IMDM-Medium in Anwesenheit von 10% hitzeinaktiviertem fötalem Kälberserum, 50 µM β-Mercaptoethanol, Standardantibiotika, und 1% IL-3 bei 37°C unter 5% CO₂ gehalten und durch Zugabe von 300 mg/l Hygromycin B (Boehringer Mannheim) selektioniert. Nach erfolgreicher Selektion wurden die Zellen langsam von IL-3 entwöhnt, der IL-3-unabhängig wachsende Zellpool anschließend in Methocel subkloniert. Einzelne Klone wurden gepickt und in Medium ohne IL-3 hochgezogen.

### iii) Testung der Brauchbarkeit der Zellen

Einzelne Klone wurden in einem Fluoreszenz-aktivierten Zell-Sorter (Becton-Dickinson, FACSort) auf Oberflächenexpression von HER2 getestet, dabei wurde als primärer Antikörper ein gegen die extrazelluläre Domäne von HER2 gerichteter Maus-Antikörper verwendet (Oncogene Science, c-neu (Ab-2)), und als sekundärer Antikörper ein Fluorescein Isothiocyanat (FITC)-konjugierter anti-Maus IgG Antikörper (Dianova, Hamburg). Der Subklon mit der besten Oberflächenexpression von HER2(V659E), genannt FDCP/Her2-C42#16, wurde für das Screenen verwendet.

### e) Herstellung von HGF (Hepatocyte Growth Factor)-Rezeptor-Testzellen

### i) Subklonierung der c-met-Sequenz in den Expressionsvektor pLXSN

Die komplette 4.6 kb große kodierende Sequenz des humanen HGF-Rezeptors c-met (GeneBank AC-Nr. J02958; Park M., et al., 1987), wurde in den mit EcoRI und XhoI doppelt geschnittenen Expressionsvektor pLXSN gerichtet kloniert. Plasmid pLXSN ist ein retroviraler Vektor, der zur Produktion rekombinanter Retroviren verwendet wird (Miller, et al. 1989). Der entstandene Expressionsvektor pLXc-met enthält die komplette kodierende Region von c-met.

### ii) Transfektion der Verpackungszellinie GP+E86

Die Verpackungszellinie GP+E86, eine Mausfibroblasten-Zellinie (Markowitz et al., 1988), wurde mit Plasmid pLXc-met folgendermaßen transfiziert: Etwa 1x10⁶ Zellen pro 10 cm Platte wurden in DMEM-Medium (Bio-Whittaker) in Anwesenheit von 10% hitzeinaktiviertem fötalem Kälberserum (FCS) in mehrere Platten ausgesät. Am nächsten Tag wurde 20 µg Plasmid-DNA mit Calciumphosphat präzipitiert und das Präzipitat langsam auf die Zellen getropft. Nach 4 Stunden wurden die Zellen für 2 min in 15% Glycerin inkubiert, gewaschen und dann in DMEM-Medium in Anwesenheit von 10% hitzeinaktiviertem fötalem Kälberserum und Standardantibiotika bei 37°C unter 5% CO₂ gehalten. Die Zellen wurden durch Zugabe von 1 g/l G418 (GIBCO BRL) selektioniert. Zellklone wurden gepoolt und der Zellpool hochgezogen.

Der Zellüberstand soll replikationsdefekte rekombinante Viruspartikel enthalten, die Zellen murinen Ursprungs infizieren. Zur Bestimmung des Virustiters wurden 5x10⁴ NIH-3T3(tk⁻) Zellen pro 6 cm Schale ausgesät und mit verschiedenen Konzentrationen des Zellüberstands (1 ml Überstand + 8 µg/ml Polybren) inkubiert. Zellen wurden in G418 selektioniert und der Virustiter aus der Zahl der Klone bestimmt.

### iii) Infektion der FDC-P1-Zellen

FDC-P1 Zellen (Dexter et al., 1980), die IL-3-abhängig wachsen, wurden folgendermaßen mit rekombinanten Viruspartikeln infiziert: Etwa 2x10⁶ Zellen wurden in 2.5 ml Virusüberstand der GP+E86/LXc-met Zellen und 8 µg/ml Polybren und 1% IL-3 für 4 Stunden bei 37°C inkubiert. Die Zellen wurden in IMDM-Medium in Anwesenheit von 10% hitzeinaktiviertem fötalem Kälberserum, 50 µM β-Mercaptoethanol, Standardantibiotika, und 1% IL-3 verdünnt und bei 37°C unter 5% CO₂ gehalten. Die Selektion erfolgte durch Zugabe von 1 g/l G418 (GIBCO BRL). Antibiotikaresistente stabile Zellinien wurden nachfolgend auf HGF-abhängiges und IL-3 unabhängiges Wachstum durch HGF-Zugabe bei IL-3 Entzug selektioniert. Dazu wurde der noch IL-3-abhängig wachsende Zellpool zuerst in Methocel subkloniert. Noch IL-3 abhängige Klone wurden gepickt und einzeln langsam von IL-3 entwöhnt, indem die Klone in 20 ng/ml rekombinantem HGF (Sigma) als Wachstumsfaktor gehalten wurden, während die IL-3 Konzentration immer weiter reduziert wurde. Einzelne HGF-abhängige jedoch IL-3-unabhängige Klone wurden hochgezogen.

### iv) Testung der Brauchbarkeit der Zellen

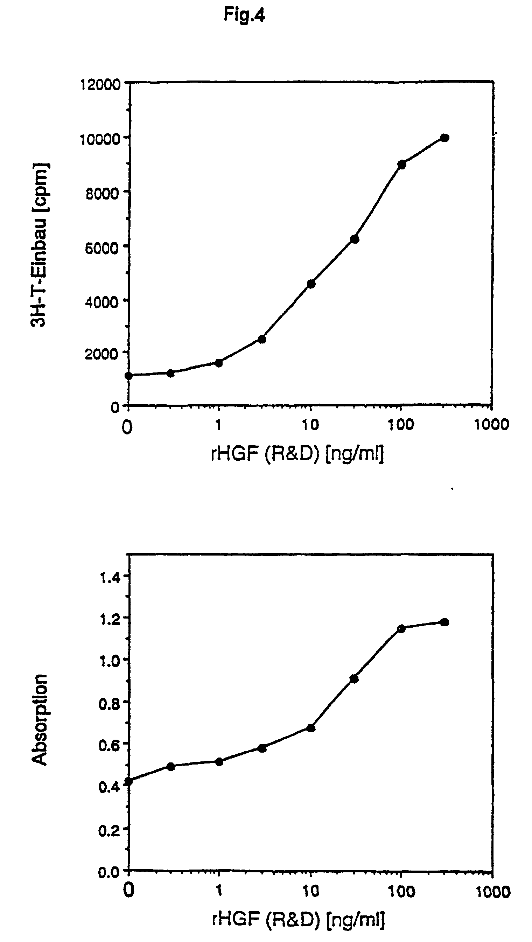

Um festzustellen, ob die Klone HGF-abhängig wachsen, wurden die Zellen in verschiedenen Konzentrationen von HGF ausgesät und das Wachstum der Zellen mittels ³H-Thymidin-Einbau bestimmt: Dazu wurden Zellen in Mikrotiterplatten ausgesät (bei einer Konzentration von 20000 Zellen pro Napf) und mit verschiedenen Konzentrationen von rekombinantem HGF (Research & Development) inkubiert. Nach 2 Tagen wurde über Nacht ³H-Thymidin zugegeben und der Thymidin-Einbau bestimmt; außerdem wurde, wie in Beispiel 1a) angegeben, die Anzahl der lebenden Zellen indirekt durch einen photometrischen MTS-Assay mittels Absorption bestimmt (Fig. 4) am nächsten Tag gemessen (Packard, TopCount). Positive Klone wurden zusätzlich in einem Fluoreszenzaktiviertem Zell-Sorter (Becton-Dickinson, FACSort) auf Oberflächenexpression von c-met getestet, dabei wurde als primärer Antikörper ein gegen die extrazelluläre Domäne von c-met gerichteter Maus-Antikörper verwendet (Upstate Biotechnology, Inc., Cat.# 05-237), und als sekundärer Antikörper ein Fluorescein Isothiocyanat (FITC)-konjugierter anti-Maus IgG Antikörper (Dianova, Hamburg). Der Subklon mit der besten Oberflächenexpression von c-met, genannt FDCP/LXc-metH6, wurde für das Screenen verwendet.

### f) High Throughput Screen

Die nach den a) bis e) erhaltenen Testzellen wurden in RPMI/1640 Medium (BioWhittaker), supplementiert mit 10% foetalem Rinderserum (FCS; Boehringer Mannheim), 2 mM Glutamin (BioWhittaker), bei 37°C und 5% CO₂, in Anwesenheit der entsprechenden Wachstumsfaktoren kultiviert. Zur Untersuchung der inhibitorischen Aktivität der zu untersuchenden Verbindungen (Testsubstanzen) wurden 1.5 x 10⁴ Zellen in 100 µl Medium pro Vertiefung in 96well Platten in oben angeführtem Medium unter Zusatz der entsprechenden Wachstumsfaktoren kultiviert. Beliebig ausgewählte Testsubstanzen lagen in einer Konzentration von 5 mg pro ml in 100% Dimethylsulfoxid (DMSO) gelöst vor und wurden in einer Verdünnung von 1:1000 den Kulturen zugefügt, wobei die DMSO Konzentration 0.1% nicht überstieg. Die Endkonzentration der Testsubstanzen betrug 5 µg/ml in dem Testsystem, jede Substanz wurde in 3 Mikrotiterplattenvertiefungen getestet (Triplikatmessung). Auf jeder Platte wurden drei Positionen nur mit DMSO in der entsprechenden Verdünnung (1:1000) versetzt; diese Positionen wurden als negative Kontrolle verwendet. Die Kulturen wurden für 21 h bei 37°C unter Zellkulturbedingungen inkubiert. Nach Ablauf der Inkubationszeit wurden 20 µl einer Mischung aus MTS (0.88 mg/ml; Promega G511A) und PMS (0.11 mg/ml; Sigma P-9625) zugegeben und weitere 3 Stunden bei 37°C inkubiert. Die Testplatten wurden nach Ablauf der Inkubationszeit in einem Mikrotiterplattenphotometer gemessen und die Daten in einer Datenbank ausgewertet. Die relative Zellzahl, die mit der Extinktion bei 492 nm korreliert, wurde in Prozent der negativen Kontrolle der Testplatte angegeben.

### g) Vergleichendes Screening und Prüfung auf Spezifität

Der in f) beschriebene High Throughput Screen wurde parallel mit den in a) bis e) beschriebenen Testzellinien durchgeführt. Dabei wurden dieselben Verdünnungen der Testsubstanz gleichzeitig auf die in verschiedenen Mikrotiterplatten vorgelegten Zellen gegeben, die in der Folge unter exakt denselben Bedingungen gehalten und ausgewertet wurden. Substanzen, die in einem Testsystem einen Wert von weniger als 50% des negativen Kontrollwerts auf der gleichen Platte kamen, wurden als potentieller Hit angesehen und näher auf Spezifität untersucht. Als spezifisch wurde eine Substanz angesehen, die in dem betreffenden Assay bei einen Wert von unter 50% der negativen Kontrolle lag und in den parallel laufenden Assays einem Wert von über 75% der entsprechenden negativen Kontrolle erreicht. Diese Substanzen wurden ausgewählt und in eine detaillierten Dosis-Wirkungskurve auf ihren IC50-Wert untersucht. Dabei wurden die Zellen in einer Mikrotiterplatte unter denselben Bedingungen, wie in f) beschrieben, vorgelegt und mit einer seriellen Verdünnung der entsprechenden Testsubstanz in Konzentrationen zwischen 400 µM und 10 nM untersucht. Als IC50 Wert wird diejenige Konzentration der Testsubstanz bezeichnet, bei der der Testwert 50% der negativen Kontrolle erreicht. Die Tabelle 1 zeigt eine Zusammenfassung der Ergebnisse, die aus Tests mit einer größeren Menge an Testsubstanzen erhalten wurden.
Eine Gesamtmenge von 231360 Tests ergab 2834 "Hits", also Substanzen, die das Kriterium "Inhibition" auf unter 50% der Kontrolle erfüllen, was einem Prozentsatz von 1.22% der getesteten Substanzen entspricht. In der Regel werden bei einschlägigen Screens in der Wirkstoff-Findung die Konzentrationen der Testsubstanzen so eingestellt, daß etwa 1-2% der Testsubstanzen die verlangten Kriterien erfüllen. Diese Substanzen müssen dann händisch und mit erheblichem Aufwand auf ihre Spezifität und den Wirkmechanismus untersucht werden. Dies geschieht in der Regel durch detaillierte Tests mit verschiedenen zellulären und enzymatischen Assays. Bei einem Screen mit hohem Durchsatz würde dies eine erheblichen Personal- und Kostenaufwand bedeuten. Die durch das vergleichende Screening gemäß der vorliegenden Erfindung gesetzten Parameter engen den Kreis der interessanten Substanzen, die in detaillierten sekundären Assays untersucht werden müssen, auf etwa 10% der ursprünglichen Hits ein. Nach detaillierten Untersuchungen blieben noch 13 Substanzen übrig (4.3% der als spezifisch eingestuften Hits), die selektiv das interessierende Zielmolekül blockieren und eine Leitstruktur für eine Wirkstoffentwicklung darstellen.

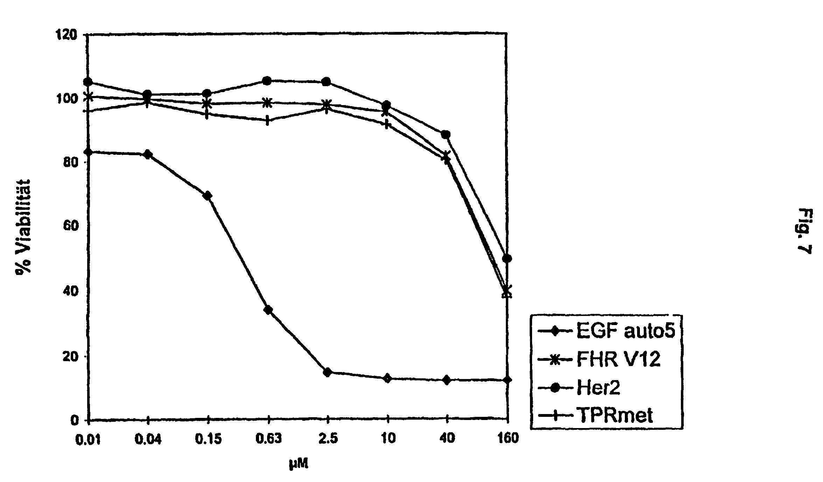

Die Spezifität eines Inhibitors für den EGF-R ist am Beispiel von 4-[(3-Chlorphenyl)amino]-6-(cyclopropylamino)-pyrimido-[5,4-d]pyrimidin in Fig. 7 dargestellt. Während der IC50-Wert für die EGFabhängige Zellinie (in Fig.7: "EGF autos") bei etwa 500 nM liegt, sind auf den anderen Testzellinien, enthaltend als Zielmoleküle Ras (in Fig.7: "FHR V12"), HER-2 bzw. den HGF-R (in Fig.7: "TPRmet"), Effekte auf die Proliferation der Zellen erst bei einer 100fach höheren Konzentration zu messen. Die Ausfallsquote von 95% nach detaillierten Tests ist dadurch zu erklären, daß die Testzellen zwar einen identischen genetischen Hintergrund haben, aber einerseits klonalen Ursprungs sind und andererseits unterschiedliche Zielmoleküle ektop exprimieren. Dadurch ergeben sich geringfügige Unterschiede in der Sensitivität gegenüber unspezifisch wirksamen organischen Substanzen.

### Beispiel 2

### Luciferase-Assay im High Throughput-Format zum Screenen von Substanzen mit spezifischer Wirkung auf Rezeptor-Tyrosinkinase-abhängige Signalübertragungswege

### a) Herstellung von rekombinanten KB-Zellen, die Luciferase in Abhängigkeit von HGF bzw. EGF exprimieren

### i) Subklonierung der c-fos Promotorregion in den Expressionsvektor pBHlucOL

Aus Plasmid pfosCAT (Schönthal, A. et al., 1988) wurde die Promotorregion des humanen c-fos Gens mit XbaI und HindIII herausgeschnitten und zunächst in Plasmid pBluescript SK (Stratagene) subkloniert. Das entstandene Zwischenprodukt wurde zuerst mit XbaI geschnitten, die Enden durch Zugabe von dNTPs und dem Klenow-Fragment der DNA-Polymerase stumpf gemacht ("blunt ending"), und dann mit SalI geschnitten. Plasmid pBHlucOL, das das Luciferasegen enthält, (Voraberger, G. et al., 1991) wurde zuerst mit HindIII geschnitten, die Enden durch Zugabe von dNTPs und dem Klenow-Fragment der DNA-Polymerase stumpf gemacht, dann mit SalI geschnitten, und hierauf das fos-Promotor Fragment hineinkloniert, resultierend in Plasmid pBHfosluci.

### ii) Transfektion der KB-Zellen

Die humane epidermoide Karzinomzellinie KB (ATCC CCL 17), wurde mit Plasmid pBHfosluci folgendermaßen transfiziert: Etwa 1x10⁶ Zellen pro 10 cm Platte wurden in DMEM-Medium (Bio-Whittaker) in Anwesenheit von 10% hitzeinaktiviertem fötalem Kälberserum in mehrere Platten ausgesät. Am nächsten Tag wurde 20 µg Plasmid-DNA mit Calcium-Phosphat präzipitiert und das Präzipitat langsam auf die Zellen getropft. Nach 4 Stunden wurden die Zellen für 2 min in 15% Glycerin inkubiert, gewaschen und dann in DMEM-Medium in Anwesenheit von 10% hitzeinaktiviertem fötalem Kälberserum und Standardantibiotika bei 37°C unter 5% CO₂ gehalten. Die Zellen wurden durch Zugabe von 0.5 g/l G418 (GIBCO BRL) selektioniert. Einzelne G418-resistente Zellklone wurden hochgezogen und auf Induzierbarkeit der Luciferaseexpression durch Zugabe von HGF (Sigma) getestet. Ungefähr 10000 Zellen jedes Klons wurden in 6facher Ausfertigung in 200 µl pro Vertiefung in einer lichtundurchlässigen, gewebekulturbeschichteten Mikrotiterplatte mit 96 Vertiefungen (Microlite™, Dynatech Laboratories) ausgesät und über Nacht bei 37°C inkubiert. Jeweils drei Ansätze wurden mit 20 ng/ml HGF behandelt und weitere 6 h inkubiert. Anschließend wurde das Medium entfernt und die Zellen zweimal mit PBS gewaschen. Die Zellen wurden in 150 µl Lyse-Puffer (25 mM Tricin, 0.5 mM EDTA, 0.54 mM Natriumtripolyphosphat, 6.5 mM DTT, 16.3 mM MgSO₄.7H₂O, 0.1% Triton X-100, 1.2 mM ATP, 0.05 mM Luciferin; pH7.8) pro Ansatz aufgenommen und die Luciferaseaktivität in einem 96well Luminometer (ML-1000, Dynatech) gemessen. Zellklon KB-B4 wurde für weitere Experimente ausgewählt, weil er einen meßbaren Basiswert an Luciferaseaktivität sowie eine 10-15-fache Induzierbarkeit durch HGF aufwies.

### iii) Testung der Brauchbarkeit der Zellinie KB-B4

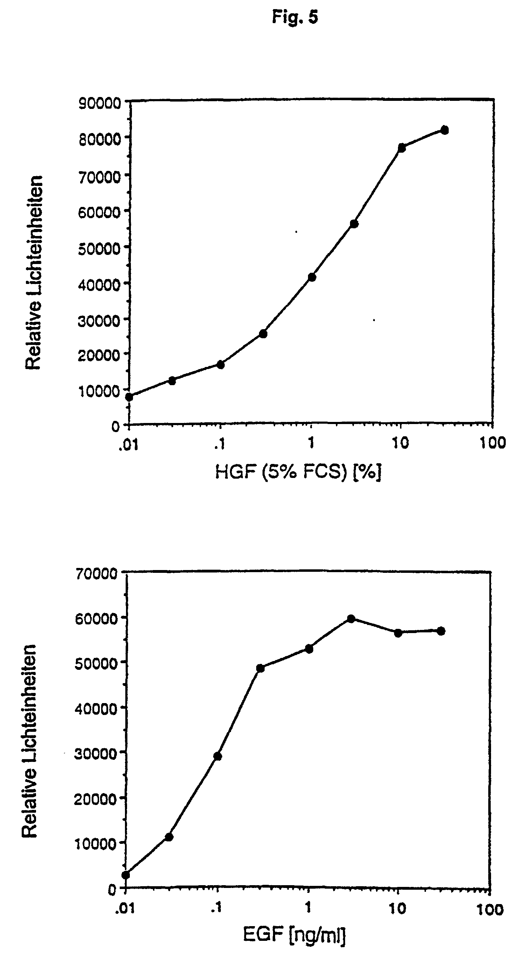

Um festzustellen, ob sich das Luciferasegen in Klon KB-B4 durch HGF bzw. EGF stimulieren läßt, wurden die Zellen in verschiedenen Konzentrationen von HGF bzw EGF ausgesät und die Luciferase-Aktivität gemessen: Dazu wurden Zellen in Mikrotiterplatten ausgesät in einer Konzentration von 10000 Zellen pro Napf und mit verschiedenen Konzentrationen HGF bzw EGF inkubiert. Nach 6 Stunden wurden die Zellen wie oben lysiert und die Luciferaseaktivität gemessen. Es konnte eine dosisabhängige Induktion gezeigt werden, mit einer maximalen Induktion von 10- bzw 20-fach. Ein Beispiel ist in Fig.5 zu sehen.

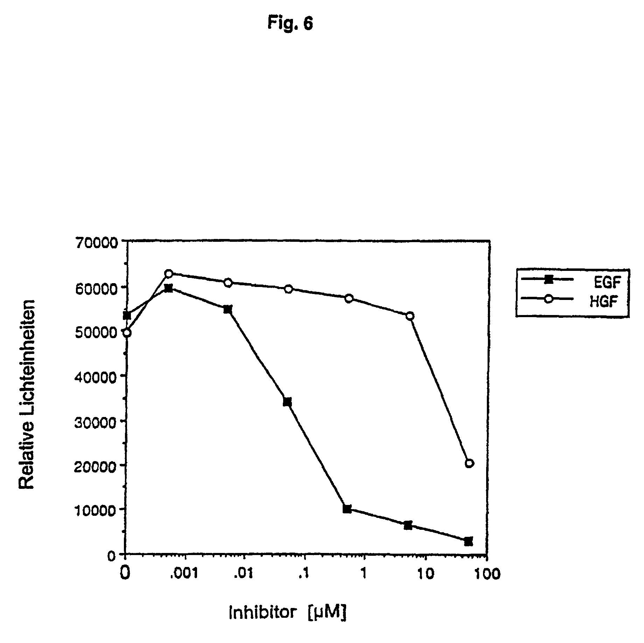

Da verschiedene Inhibitoren der Tyrosinkinase Aktivität des EGF-Rezeptors bekannt sind, wurden KB-B4 Zellen mit verschiedenen Konzentrationen unterschiedlicher Inhibitoren unter Zugabe von 1 ng/ml EGF 6 h lang inkubiert und dann die Luciferaseaktivität gemessen. Mit allen getesteten Inhibitoren konnte eine Dosisabhängige Inhibierung der Luciferase-Induktion durch EGF erreicht werden. Ein Beispiel für die inhibitorische Wirkung eines Inhibitors ist, am Beispiel der Testsubstanz 6,7-Dimethoxy-4-(3-methylphenylamino)chinazolin, in Fig.6 zu sehen.

### b) High Throughput Screen

Die in a) beschriebenen KB4-Zellen zum Test auf EGF und HGF wurden in DMEM (Bio Whittaker), supplementiert mit 10% FCS und 500 mg/ml G418, propagiert. Zur Untersuchung der Testsubstanzen wurden 1 x 10⁴ Zellen in 100 µl phenolrotfreiem DMEM/1% FCS pro Vertiefung in 96 Well Mikrotiterplatten ausgesät. Beliebig ausgewählte Testsubstanzen lagen in einer Konzentration von 5 mg pro ml in 100% Dimethylsulfoxid (DMSO) gelöst vor und wurden in einer Verdünnung von 1:600 den Kulturen zugefügt, wobei die DMSO Konzentration 0.25% nicht überstieg. Die Endkonzentration der Testsubstanzen betrug ca. 8.3 µg/ml in dem Testsystem, jede Substanz wurde in drei Mikrotiterplattenvertiefungen getestet (Triplikatmessung). Auf jeder Platte wurden drei Positionen nur mit DMSO in der entsprechenden Verdünnung (1:600) versetzt; diese Positionen wurden als negative Kontrolle verwendet. Nach Zugabe der Testsubstanzen wurden die Testzellen zur Induktion des fos-Promotors mit EGF (20 ng/ml) bzw. HGF (20 ng/ml) induziert. Nach einer Inkubationszeit von 5 h bei 37°C und 5% CO₂ wurden 150 µl Lysepuffer (25 mM Tricin, 0.5 mM EDTA, 0.54 Natriumtripolyphosphat, 16.3 mM MgSO₄, 1.2 mM ATP, 0.05 mM Luciferin, 56.8 mM β-Mercaptoethanol, 0.1% Triton X-100, pH 7.8) zugegeben und die Lumineszenz in einem Wallac Microbeta gemessen und die Daten in einer Datenbank ausgewertet. Die relative Promotoraktivität, die mit der gemessenen Lumineszenz korreliert, wurde in Prozent der negativen Kontrolle der Testplatte angegeben.

### c) Vergleichendes Screening und Prüfung auf Spezifität

Der in b) beschriebene High Throughput Screen wurde parallel mit den zwei oben genannten Zellinien durchgeführt. Dabei wurden dieselben Verdünnungen der Testsubstanz gleichzeitig auf die in verschiedenen Mikrotiterplatten vorgelegten Zellen gegeben, die in der Folge unter exakt denselben Bedingungen gehalten und ausgewertet wurden. Substanzen, deren Ergebnis in dem einem Testsystem weniger als 50% der negativen Kontrolle und in dem parallelen Assay mehr als 70% der negativen Kontrolle betrug, wurden als spezifische Inhibitoren des betreffenden Testsystems angesehen. Solche Substanzen wurden zur Überprüfung der Reproduzierbarkeit ein zweites Mal in einem entsprechend durchgeführten High Throughput Screen getestet. Substanzen, deren Ergebnisse wiederholbar waren, wurden in einer detaillierten Dosis-Wirkungskurve auf ihren IC-50 Wert untersucht. Dabei wurden die Zellen in einer Mikrotiterplatte unter gleichen Bedingungen unter b) beschrieben vorgelegt und mit einer seriellen Verdünnung der entsprechenden Testsubstanz in Konzentrationen zwischen 400 uM und 10 nM untersucht. Als IC50 Wert wird diejenige Konzentration der Testsubstanz bezeichnet, bei der der Testwert 50% der negativen Kontrolle erreicht. Tab. 1 zeigt eine Zusammenfassung der Ergebnisse, die aus Tests mit 2676 Substanzen erhalten wurden.

**Tab. 1**

| Assay | Hits | Hits % | spezifi= sche Hits | spezifische Hits in % |
|---|---|---|---|---|
| EGF | 28 | 1.04 | 7 | 0.26 |
| HGF | 27 | 1.00 | 6 | 0.22 |

### Beispiel 3

### Luciferase-Assay im High Throughput-Format zum Screenen von Substanzen mit spezifischer Wirkung auf Signalübertragungswege, die abhängig sind von G-Protein-gekoppelten Rezeptoren

### a) Herstellung von drei verschiedenen rekombinanten A549 Test-Zellinien, die Luciferase in Abhängigkeit von der Aktivierung des humanen Neurokinin-1(NK1)-Rezeptors, des Neurokinin-2(NK2)-Rezeptors bzw. des Serotoninrezeptors 5HT₂-exprimieren

Die Herstellung sowie die Kultivierung der NK2-Zellinie (A20/NK2-122) und der 5HT₂-Zellinie wurden durchgeführt, wie in Beispiel 6 der WO 93/11257 beschrieben. Zur Herstellung der NK1-Zellinie (A20/NK1-12A) wurde die cDNA des humanen NK1-Rezeptors (Gerard et al., 1991) analog zu NK2 in den Expressionsvektor pAHygCMV1 kloniert und damit die Prätest-Zellinie transfiziert. Das weitere Vorgehen sowie die Kultivierung erfolgten entsprechend den NK2-Testzellen.

### b) High Throughput Screen

Die in a) hergestellten Zellinien zum Test auf NK1 und NK2 werden in RPMI/1640 Medium (BioWhittaker), supplementiert mit 10% dialysiertem FCS, 0.8 g/l G418 und 0.15 g/l Hygromycin, propagiert. Zur Untersuchung der Testsubstanzen werden 1x10⁴ Zellen in 100 µl UltraCulture Medium (BioWhittaker) pro Vertiefung in 96 Well Mikrotiterplatten ausgesät. Beliebig ausgewählte Testsubstanzen lagen in einer Konzentration von 5mg/ml in 100% Dimethylsulfoxid (DMSO) gelöst vor. Sie wurden in zwei Konzentrationen von 1:300 und 1:3000 den Kulturen zugefügt, wobei die DMSO Konzentration 0.5% nicht überstieg. Die Endkonzentration der Testsubanzen betrug etwa 16.6 und 1.66 µg/ml in dem Testsystem, jede Substanz wurde pro Verdünnung in vier Mikrotiterplattenvertiefungen getestet. Auf jeder Platte wurden vier Positionen pro Verdünnung nur mit DMSO in der entsprechenden Verdünnung (1:300, 1:3000) versetzt. Diese Positionen wurden als negative Kontrolle verwendet. Nach Zugabe der Testsubstanzen wurden die NK1-Zellen zur Induktion der TRE-Sensor-DNA mit Substanz P (1 µM), die NK2-Zellen mit Neurokinin A (10 µM) und die 5HT₂-Zellen mit Serotonin (100 µM) versetzt. Nach einer Inkubationszeit von 7 h bei 37°C und 5% CO₂ wurden die Mikrotiterplatten 4 mal mit je 150 µl Waschpuffer pro Vertiefung gewaschen (25 mM Tricin, 16.3 mM MgSO₄). Anschließend wurden 100 µl Lysepuffer (25 mM Tricin, 0.5 mM EDTA, 0.54 mM Natriumtripolyphosphat, 16.3 mM MgSO₄, 1.2 mM ATP, 0.05 mM Luciferin, 56.8 mM β-Mercaptoethanol, 0.1% Triton X-100, pH 7.8)zugegeben und die Lumineszenz in einem Dynatech Luminometer gemessen und die Daten in einer Datenbank ausgewertet. Die relative Promotoraktivität, die mit der gemessenen Lumineszenz korreliert, wurde in Prozent der negativen Kontrolle der Testplatte angegeben.

### c) Vergleichendes Screening und Prüfung auf Spezifität

Der in b) beschriebene High Throughput Screen wurde parallel mit den drei oben genannten Zellinien durchgeführt. Dabei wurden dieselben Verdünnungen der Testsubstanzen gleichzeitig auf die in verschiedenen Mikrotiterplatten vorgelegten Zellen gegeben, die in der Folge unter exakt denselben Bedingungen gehalten und ausgewertet wurden. Substanzen, deren Ergebnis in einem Testsystem weniger als 40% der negativen Kontrolle und in den anderen parallelen Assays mehr als 65% der negativen Kontrolle betrug, wurden als spezifische Inhibitoren des betreffenden Testsystems angesehen. Solche Substanzen wurden zur Überprüfung der Reproduzierbarkeit ein zweites Mal in einem entsprechend durchgeführten High Throughput Screen getestet. Substanzen, deren Ergebnisse wiederholbar waren, wurden ausgewählt und weiteren Folgetests unterworfen. Einige Substanzen hemmen auch zwei der drei Testsysteme, einige hemmen unspezifisch alle drei parallel getesteten Assays. Tab. 2 zeigt eine Zusammenfassung der Wirkung von 1458 Substanzen auf die beschriebenen Tests.

**Tab. 2**

| Assay | Anzahl der Hits | in % |
|---|---|---|
| nur NK1, nicht NK2, 5HT2 | 5 | 0.34 |
| nur NK2, nicht NK1, 5HT2 | 18 | 1.23 |
| NK1 und NK2, nicht 5HT2 | 6 | 0.41 |
| nur 5HT2, nicht NK1. NK2 | 74 | 5.07 |
| NK1, NK2 und 5HT2 | 61 | 4.18 |

### Literatur

Alexander, W. S., et al., 1991, EMBO J. 10, 3683-3691
Bos, J. L., 1988, Mutation Res. 195, 255-271
Brasier, A. R., et al., 1989, BioTechniques 7, 1116-1122
Chalfie, M., et al., 1994, Science 263, 802-805
Coussens, L., et al., 1985, Science 230, 1132-1139
Daley, G. Q., et al., 1990, Science 247, 824-830
De Wet, J. R., et al., 1987, Mol. Cell. Biol. 7, 725-737
Dexter, T. M., et al., 1980, J. Exp. Med. 152, 1036-1047
Felgner, P. L., et al., 1987, Proc.Natl.Acad.Sci. USA 84, 7413-7417
Fiebich, B. L., et al., 1993, Eur.J.Biochem. 211, 19-26
Furth, M. E., et al., 1982, J. Virol. 43, 294-304
Gerard, N. P., et al., 1991, Biochemistry 30 (44), 10640-10646
Hartmann, A., 1991, BioTec 5, 40-45
Hudziak, R.M., et al., 1987, Proc. Natl. Acad. Sci. USA 84, 7159-7163
Inducible Gene Expression, 1995, Vol.I/II, Baeuerle, P. A., et al., Birkhäuser (Boston, Basel, Berlin), ISBN 0-8176 37281, ISBN 0-8176 37346
Inouye, S. und Tsuji, F. I., 1994, FEBS Lett. 341, 277-280
James, G. L., et al., 1995, J. Biol. Chem. 270, 6221-6226
Karasuyama,H. und Melchers, F., 1988, Eur. J. Immunol. 18, 97-104
Kiaris, H. und Spandios, D. A., 1995, Int. J. Oncol. 7, 413-421
Kitada, S., et al., 1994, Antisense Research and Development 4, 71-79
Kohl, N. E., et al., 1993, Science 260, 1934-1937
Kohl, N. E., et al., 1994, Proc. Natl. Acad. Sci. USA 91, 9141-9145
Kohl, N. E., et al., 1995, Nature Med. 1, 792-797
Kricka, L.J., 1988, Analyt. Biochem. 175, 14-21
Lerner, E. C., et al., 1995, J. Biol. Chem. 270, 26770-26773
Lu, Q.-L., et al., 1993, Int. J. Cancer 53, 29-35
Manne, V., et al., 1995, Oncogene 10, 1763-1779
Markowitz, D., et al., 1988, J. Virol. 62, 1120-1124
Matthews, W., et al., 1991, Proc. Natl. Acad. Sci. USA 88, 9026-9030; GenBank Accession Nr. X59397
McCoy, M. S., et al., 1984, Mol. Cell. Biol. 8, 1577-1582
McDonnell, T., et al., 1992, Cancer Res. 52, 6940-6944
Meckling-Gill, K. A., et al., 1992, Biochem. Biophys. Acta 1137, 65-72
Metcalf, D., et al., 1992, Proc. Natl. Acad. Sci. USA 89, 2819-2823
Miyashita, T. und Reed, J. C., 1992, Cancer Res. 52, 5407-5411
Miyashita, T. et al., 1994, Oncogene 9, 1799-1805
Millauer, et al., 1993, EMBL Accession Nr. X70842
Miller, A. D. et al., 1989, Bio Techniques 7, 980-990
Nunez, G., et al., 1990, J. Immunol. 144, 3602-3610
Park, M.,et al.,1987, Proc. Natl. Acad. Sci. U.S.A. 84, 6379-6383
Pezzella, F., et al., 1993, N. Engl. J. Med 329, 690-694
Pierce, J. H. et al., 1988, Science 239, 628-631
Potter, H., Weir, L., und Leder, P., 1984, Proc.Natl.Acad.Sci. USA 81, 7161.
Quilliam, L. A., et al., 1994, Mol. Cell. Biol. 14, 1113-1121
Rapp, U. R., et al., 1985, Nature 317, 434-438
Reddy, E. P., et al., 1982, Nature 300, 149-152
Reed, J. C., 1994, J. of Cell Biology 124, 1&2, 1-6
von Rüden, T., et al., 1988, EMBO J. 7, 2749-2756
Schönthal A., et al., 1988, Cell 54, 325-334
Shibuya, H., et al., 1992, Cell 70, 57-67
Stacey, D. W., et al., 1991, Mol. Cell. Biol. 11, 4053-4064
Subramani, S. und DeLuca, M., 1987, Genetic Engineering, Principles and Methods, J.K. Sedlow ed., Plenum Press, New York, Band 10, 75-89
Suda, Y., et al., 1990, EMBO J. 9, 181-190
Tanaka, N., et al., 1994, Cell 77, 829-839
Terman et al., 1992, Biochem. Biophys. Res. Commun. 187, 1579-1586; EMBL Accession Nr. X61656
Ullrich, A., et al.,1984, Nature 309, 418-425
Voraberger G., et al., 1991, J. Immunol. 147, 2777-2786
Weiner, D. B., et al., 1989, Nature 339, 230-231
Wieland, E., et al., 1985, Ärztl. Lab. 31, 203-214

## Patentansprüche

1. Verfahren zum Bestimmen der pharmakologischen Wirkung einer Substanz auf die Aktivität biologischer Zielproteine, wobei man eine Substanz auf Testzellen aufbringt, die ein oder mehrere biologische Zielproteine enthalten und die Wirkung der Substanz auf die Aktivität der Zielproteine bestimmt, **dadurch gekennzeichnet, daß** man eine definierte Menge einer Testsubstanz in einem Arbeitschritt
a) auf Testzellen aufbringt, die klonale Zellen sind, wobei sich die Zellen hinsichtlich eines oder mehrerer biologischer Zielproteine unterscheiden; und/oder
b) auf Testzellen aufbringt, die ein oder mehrere identische Zielproteine enthalten, wobei sich die Zellen dadurch unterscheiden, daß sie unterschiedlichen Typs oder Zellen desselben Typs mit unterschiedlichem Differenzierungsoder Aktivierungszustand sind, und daß man
i) die Wirkung der Substanz auf das bzw. die biologischen Zielproteine mittels eines an die Aktivierung des Zielproteins gekoppelten Nachweissystems mißt; und/oder
ii) die Wirkung der Substanz auf unterschiedliche durch die Aktivierung des Zielproteins ausgelöste Regulationsmechanismen bestimmt, indem man die Wirkung mittels mehrerer, jeweils an die unterschiedlichen Regulationsmechanismen gekoppelten Nachweissysteme mißt,
und daß man die Wirkungen der Testsubstanz auf die unterschiedlichen Testzellen bzw. die mittels unterschiedlicher Nachweismethoden ermittelten Wirkungen unmittelbar miteinander vergleicht.

2. Verfahren nach Anspruch 1, **dadurch gekennzeichnet, daß** man mehrere Substanzen, gegebenenfalls jeweils in mehreren Verdünnungen, parallel auf eine oder mehrere Batterien von zellulären Substraten, wobei jede Batterie eine Gruppe unterschiedlicher Assays bzw. Assay-Formate auf der Grundlage derselben Ausgangszelle darstellen, aufbringt.

3. Verfahren nach Anspruch 1 oder 2, **dadurch gekennzeichnet, daß** die Testzellen Säugetierzellen sind.

4. Verfahren nach Anspruch 3, **dadurch gekennzeichnet, daß** die Testzellen humane Zellen sind.

5. Verfahren nach einem der vorhergehenden Ansprüche, **dadurch gekennzeichnet, daß** die Testzellen das interessierende Zielprotein endogen exprimieren.

6. Verfahren nach einem der Ansprüche 1 bis 4, **dadurch gekennzeichnet, daß** die Testzellen mit der für das interessierende Zielprotein kodierenden DNA transformiert sind.

7. Verfahren nach einem der vorhergehenden Ansprüche, **dadurch gekennzeichnet, daß** man die Substanz außerdem auf Kontrollzellen aufbringt, die das interessierende Zielprotein nicht enthalten und daß man die Wirkung der Testsubstanz auf die Testzellen mit ihrer Wirkung auf die Kontrollzellen vergleicht.

8. Verfahren nach einem der vorhergehenden Ansprüche, **dadurch gekennzeichnet, daß** das Zielprotein ein Rezeptor ist.

9. Verfahren nach Anspruch 8, **dadurch gekennzeichnet, daß** der Rezeptor eine Tyrosinkinase ist.

10. Verfahren nach Anspruch 9, **dadurch gekennzeichnet, daß** der Rezeptor der EGF-Rezeptor ist.

11. Verfahren nach Anspruch 9, **dadurch gekennzeichnet, daß** der Rezeptor HER2 ist.

12. Verfahren nach Anspruch 9, **dadurch gekennzeichnet, daß** der Rezeptor der HGF-Rezeptor ist.

13. Verfahren nach Anspruch 9, **dadurch gekennzeichnet, daß** der Rezeptor KDR ist.

14. Verfahren nach Anspruch 8, **dadurch gekennzeichnet, daß** Rezeptor ein G-Protein-gekoppelter Rezeptor ist.

15. Verfahren nach Anspruch 14, **dadurch gekennzeichnet, daß** der Rezeptor ein Neurokinin-Rezeptor ist.

16. Verfahren nach Anspruch 14, **dadurch gekennzeichnet, daß** der Rezeptor ein Serotonin-Rezeptor ist.

17. Verfahren nach einem der Ansprüche 1 bis 7, **dadurch gekennzeichnet, daß** das interessierende Zielprotein eine intrazelluläre Komponente eines Signalübertragungsweges ist.

18. Verfahren nach Anspruch 17, **dadurch gekennzeichnet, daß** das Zielprotein eine Proteinkinase ist.

19. Verfahren nach Anspruch 17, **dadurch gekennzeichnet, daß** das Zielprotein ras ist.

20. Verfahren nach Anspruch 17, **dadurch gekennzeichnet, daß** das Zielprotein raf ist.

21. Verfahren nach einem der Ansprüche 1 bis 7, **dadurch gekennzeichnet, daß** das interessierende Zielprotein ein an der Apoptose beteiligtes Molekül ist

22. Verfahren nach Anspruch 21, **dadurch gekennzeichnet, daß** das Zielprotein bcl-2 ist.

23. Verfahren nach Anspruch 1, **dadurch gekennzeichnet, daß** die Testzellen ein Reportergen unter der Kontrolle einer regulatorischen Sequenz, die auf die Änderung der Konzentration eines Botenstoffes eines Signalübertragungsweges anspricht, von dem das interessierende Zielprotein eine Komponente ist, enthalten, und daß man die Wirkung der Testsubstanz auf das Zielprotein in einer Änderung der Expression des Reportergens bestimmt.

24. Verfahren nach Anspruch 23, **dadurch gekennzeichnet, daß** das Reportergen Luciferase ist.

25. Verfahren nach Anspruch 23, **dadurch gekennzeichnet, daß** das Reportergen das Green Fluorescent Protein ist.

26. Verfahren nach Anspruch 1, **dadurch gekennzeichnet, daß** man Testzellen, die hinsichtlich ihrer Proliferation abhängig von einem Wachstumsfaktor sind, in Gegenwart des Wachstumsfaktors kultiviert und daß man die Wirkung der Substanz auf die Zellen bestimmt, indem man direkt oder indirekt die Apoptose oder die Proliferation der Zellen mißt.

27. Verfahren nach einem der vorhergehenden Ansprüche, **dadurch gekennzeichnet, daß** man es im High Throughput Format durchführt.

## Claims

1. Process for determining the pharmacological effect of a substance on the activity of biological target proteins, wherein a substance is applied to test cells which contain one or more biological target proteins and the effect of the substance on the activity of the target proteins is determined, **characterised in that** in one operational step a defined amount of a test substance
a) is applied to test cells which are clonal cells, which differ with regard to one or more biological target proteins; and/or
b) is applied to test cells which contain one or more identical target proteins, the cells differing **in that** they are of different types or are cells of the same type with different states of differentiation or activation, and **in that**
i) the effect of the substance on the or each biological target protein is measured using a detection system coupled to the activation of the target protein; and/or
ii) the effect of the substance on different regulatory mechanisms triggered by the activation of the target protein is determined by measuring the effect using a plurality of detection systems each coupled to the different regulatory mechanisms,
and the effects of the test substance on the different test cells or the effects determined using different detection methods are directly compared with one another.

2. Process according to claim 1, **characterised in that** a plurality of substances, optionally in several dilutions, are applied in parallel to one or more sets of cellular substrates, each set constituting a group of different assays or assay formats based on the same starting cell.

3. Process according to claim 1 or 2, **characterised in that** the test cells are mammalian cells.

4. Process according to claim 3, **characterised in that** the test cells are human cells.

5. Process according to one of the preceding claims, **characterised in that** the test cells endogenously express the target protein of interest.

6. Process according to one of claims 1 to 4, **characterised in that** the test cells are transformed with the DNA coding for the target protein of interest.

7. Process according to one of the preceding claims, **characterised in that** the substance is also applied to control cells which do not contain the target protein of interest and the effect of the test substance on the test cells is compared with their effect on the control cells.

8. Process according to one of the preceding claims, **characterised in that** the target protein is a receptor.

9. Process according to claim 8, **characterised in that** the receptor is a tyrosine kinase.

10. Process according to claim 9, **characterised in that** the receptor is the EGF receptor.

11. Process according to claim 9, **characterised in that** the receptor is HER2.

12. Process according to claim 9, **characterised in that** the receptor is the HGF receptor.

13. Process according to claim 9, **characterised in that** the receptor is KDR.

14. Process according to claim 8, **characterised in that** the receptor is a G protein-coupled receptor.

15. Process according to claim 14, **characterised in that** the receptor is a neurokinin receptor.

16. Process according to claim 14, **characterised in that** the receptor is a serotonin receptor.

17. Process according to one of claims 1 to 7, **characterised in that** the target protein of interest is an intracellular component of a signal transmission pathway.

18. Process according to claim 17, **characterised in that** the target protein is a protein kinase.

19. Process according to claim 17, **characterised in that** the target protein is ras.

20. Process according to claim 17, **characterised in that** the target protein is raf.

21. Process according to one of claims 1 to 7, **characterised in that** the target protein of interest is a molecule which participates in apoptosis.

22. Process according to claim 21, **characterised in that** the target protein is bcl-2.

23. Process according to claim 1, **characterised in that** the test cells contain a reporter gene under the control of a regulatory sequence which responds to the change in the concentration of a messenger substance of a signal transmission pathway, of which the target protein of interest is a component, and that the effect of the test substance on the target protein is determined in a change in the expression of the reporter gene.

24. Process according to claim 23, **characterised in that** the reporter gene is luciferase.

25. Process according to claim 23, **characterised in that** the reporter gene is Green Fluorescent Protein.

26. Process according to claim 1, **characterised in that** test cells which are dependent on a growth factor for their proliferation are cultivated in the presence of the growth factor and the effect of the substance on the cells is determined by directly or indirectly measuring the apoptosis or the proliferation of the cells.

27. Process according to one of the preceding claims, **characterised in that** it is carried out in the High Throughput Format.

## Revendications

1. Procédé pour déterminer l'effet pharmacologique d'une substance sur l'activité de protéines cibles biologiques, où l'on applique une substance sur des cellules tests qui contiennent une ou plusieurs protéines cibles biologiques et on détermine l'effet de la substance sur l'activité dès protéines cibles, **caractérisé en ce que** l'on applique une quantité définie d'une substance test dans une étape opératoire
a) sur des cellules tests qui sont des cellules clonales, les cellules différant concernant une ou plusieurs protéines cibles biologiques ; et/ou
b) sur des cellules tests qui contiennent une ou plusieurs protéines cibles identiques, les cellules différant **en ce qu'**elles sont de type différent ou des cellules du même type dans un état de différenciation ou d'activation différent, et **en ce que**
i) l'on mesure l'effet de la substance sur la ou les protéines cibles biologiques au moyen d'un système de mise en évidence couplé à l'activation de la protéine cible ; et/ou
ii) l'on détermine l'effet de la substance sur différents mécanismes de régulation déclenchés par l'activation de la protéine cible, en mesurant l'effet au moyen de plusieurs systèmes de mise en évidence couplés à chaque fois aux différents mécanismes de régulation,
et **en ce que** l'on compare directement entre eux les effets de la substance test sur les différentes cellules tests ou les effets déterminés au moyen de différentes méthodes de mise en évidence.

2. Procédé selon la revendication 1 **caractérisé en ce que** l'on applique plusieurs substances, éventuellement à chaque fois en plusieurs dilutions, parallèlement sur une ou plusieurs batteries de substrats cellulaires, chaque batterie représentant un groupe d'essais ou de formats d'essais différents sur la base de la même cellule initiale.

3. Procédé selon la revendication 1 ou 2 **caractérisé en ce que** les cellules tests sont des cellules de mammifère.

4. Procédé selon la revendication 3 **caractérisé en ce que** les cellules tests sont des cellules humaines.

5. Procédé selon l'une des revendications précédentes **caractérisé en ce que** les cellules tests expriment de manière endogène la protéine cible intéressante.

6. Procédé selon l'une des revendications 1 à 4 **caractérisé en ce que** les cellules tests sont transformées avec l'ADN codant la protéine cible intéressante.

7. Procédé selon l'une des revendications précédentes **caractérisé en ce que** l'on applique en outre la substance sur des cellules témoins qui ne contiennent pas la protéine cible intéressante et en çe que l'on compare l'effet de la substance test sur les cellules tests avec son effet sur les cellules témoins.

8. Procédé selon l'une des revendications précédentes **caractérisé en ce que** la protéine cible est un récepteur.

9. Procédé selon la revendication 8 **caractérisé en ce que** le récepteur est une tyrosine kinase.

10. Procédé selon la revendication 9 **caractérisé en ce que** le récepteur est le récepteur de EGF.

11. Procédé selon la revendication 9 **caractérisé en ce que** le récepteur est HER2.

12. Procédé selon la revendication 9 **caractérisé en ce que** le récepteur est le récepteur de HGF.

13. Procédé selon la revendication 9 **caractérisé en ce que** le récepteur est KDR.

14. Procédé selon la revendication 8 **caractérisé en ce que** le récepteur est un récepteur couplé à une protéine G.

15. Procédé selon la revendication 14 **caractérisé en ce que** le récepteur est un récepteur de neurokinine.

16. Procédé selon la revendication 14 **caractérisé en ce que** le récepteur est un récepteur de sérotonine.

17. Procédé selon l'une des revendications 1 à 7 **caractérisé en ce que** la protéine cible intéressante est un composant intracellulaire d'une voie de transmission de signaux.

18. Procédé selon la revendication 17 **caractérisé en ce que** la protéine cible est une protéine kinase.

19. Procédé selon la revendication 17 **caractérisé en ce que** la protéine cible est ras.

20. Procédé selon la revendication 17 **caractérisé en ce que** la protéine cible est raf.

21. Procédé selon l'une des revendications 1 à 7 **caractérisé en ce que** la protéine cible intéressante est une molécule impliquée dans l'apoptose.

22. Procédé selon la revendication 21 **caractérisé en ce que** la protéine cible est bcl-2.

23. Procédé selon la revendication 1 **caractérisé en ce que** les cellules tests contiennent un gène reporter sous le contrôle d'une séquence régulatrice qui réagit à la variation de la concentration d'un messager d'une voie de transmission de signaux dont la protéine cible intéressante est un composant, et **en ce que** l'on détermine l'effet de la substance test sur la protéine cible par le biais d'une modification de l'expression du gène reporter.

24. Procédé selon la revendication 23 **caractérisé en ce que** le gène reporter est la luciférase.

25. Procédé selon la revendication 23 **caractérisé en ce que** le gène reporter est la Green Fluorescent Protein.

26. Procédé selon la revendication 1 **caractérisé en ce que** l'on cultive des cellules tests qui, concernant leur prolifération, dépendent d'un facteur de croissance, en présence du facteur de croissance et **en ce que** l'on détermine l'effet de la substance sur les cellules en mesurant directement ou indirectement l'apoptose ou la prolifération des cellules.

27. Procédé selon l'une des revendications précédentes **caractérisé en ce qu'**il est mis en oeuvre dans le High Throughput Format.
